# EUROPEAN PATENT APPLICATION

(11) **EP 2 388 250 A1**
(43) Date of publication of application: **23.11.2011**
(21) Application number: 11176669.7
(22) Date of filing: 13.10.2006
(51) Int. Cl.: C07D 239/56, A61K 31/513, A61P 31/18

(54) **4-Pyrimidinone derivatives and their use as anti-HIV agents**

(30) Priority: 13.10.2005 IT RM20050512
(62) Divisional of application: 06810026.2
(71) Applicant: UNIVERSITA DEGLI STUDI DI SIENA, 53100 Siena (IT)
(72) Inventor: Botta, Maurizio, 53100 SIENA (IT); Corelli, Federico, 53100 SIENA (IT); Petricci, Elena, 53100 SIENA (IT); Radi, Marco, 53100 SIENA (IT); Maga, Giovanni, 27100 PAVIA (IT); Mai, Antonello, 00137 Roma (IT)
(74) Representative: Capasso, Olga

(57) **Abstract**

The invention relates to 4-pyrimidinone derivatives of formula V and the use thereof as medicaments in particular for the treatment of HIV infections, the use thereof for preparing pharmaceutical compositions and methods for the preparation thereof. 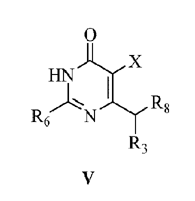 R₆ is -Y-R₇ or NR₄R₅; Y = S, SO, SO₂; R₃ is aryl or aralkyl; R₈ is methyl.

## Description

### BACKGROUND TO THE INVENTION

The invention relates to 6-vinyl pyrimidine pyrimidinone derivatives and the use thereof as medicament in particular for the treatment of HIV infections, the use thereof for preparing pharmaceutical compositions and methods for the preparation thereof.

### PRIOR ART

Acquired immunodeficiency syndrome (AIDS) is a disease of viral origin for which so far no appropriate therapy has been developed. More than twenty years have passed since the US Center for Disease Control (CDC) reported AIDS for the first time (Gallo, R. C. Science 2002, 298, 1728.); a few years later HIV was identified as the aetiological agent of this pathology. Within a short space of time, AIDS assumed epidemic proportions throughout the world, to the point that today more than 40 million people are infected with AIDS and more than 3 million deaths from this disease were reported in 2003 alone (WHO (UNAIDS), June **2004**).

In 1996, the development of highly active antiretroviral therapy (HAART) and the use of a cocktail of drugs had a significant impact on both the treatment of patients and the development of the disease in various countries. Nevertheless, most people infected by HIV/AIDS do not have access to antiretroviral therapy and to the most common chemotherapies used due to the prohibitive costs (Fauci, A. S. Nat. Med. 2003, 9, 839.).

HIV belongs to the class of retroviruses, i.e. those viruses in which the genetic information is carried by the RNA (Turner, B. G.; Summers, M. F. J. Mol. Biol. 1999, 285, 1.). HIV infects the T cells that carry the antigen CD4 on the surface. In particular, the virus infection requires the fusion of the viral and cellular membranes; this process is mediated by the viral glycoproteins of the capside (gp 120, gp41) and by the receptors (CD4 and coreceptors, such as CCR5 or CXCR4) of the target cell. When the virus enters the cell, the RNA thereof is reverse-transcribed to DNA through a viral enzyme, reverse transcriptease (RT). The viral DNA is then integrated into the DNA of the host cell through the entry of HIV. Activation of the host cell is translated into transcriptease of the viral DNA in m-RNA, which is in turn transformed into viral glycoproteins. The protease of HIV, the third viral enzyme, occurs in the phase in which the precursors of the viral glycoproteins are broken down into mature single proteins.

RNA and the viral glycoproteins assemble at the level of the cell surface to form new virions that are released outside the cell to infect other cells. The extensive cell damage arising from the destruction of the host's genetic material and from the release of the virions leads to the death of the infected cells.

There currently exist three classes of antiretroviral drugs approved by the FDA for the treatment of HIV/AIDS. These drugs are reverse transcriptease inhibitors (RTIs), protease inhibitors (PIs) and, recently, entry inhibitors.

The RTIs can be further subdivided into nucleoside inhibitors (NRTIs) and non nucleoside reverse transcriptease inhibitors (NNRTIs). The NRTIs are simply modified nucleosides without the hydroxy group in position 3' which, once the nucleosides have been incorporated into the DNA, entail the termination of the transcription of the DNA. The anti-HIV activity of these compounds depends on the intracellular phosphorylation thereof and on the capacity of the phosphorylated molecules to interact with the RT of HIV-1. The greater limits to use of the similar nucleosides are due to the toxicity, to the early development of resistance by the virus. The non nucleoside RT inhibitors (NNRTIs) bind at a allosteric site far from the polymerisation site, causing a conformational variation of the active site of the RT that translates into inhibition of the action thereof. The NNRTIs can be further distinguished from the NRTIs by the characteristic resistance to mutations and lack of activity in relegation to HIV-2. On the basis of current knowledge, two strains of HIV are known: HIV-1 and HIV-2. The former is found mainly in Europe, America and Central Africa. HIV-2, on the other hand, is found more commonly in West Africa and Asia and causes a milder clinical syndrome than the former strain. Currently, there are no drugs approved by the FDA for treating HIV-2 and the diagnostic test used for HIV-1 is not applicable to HIV-2 and it is very difficult to isolate it from the blood. In addition, HIV-2 infections develop more slowly. The infections are often asymptomatic and as much as 15-20 years may pass before the infection manifests itself in the blood. The transmission paths are the same as for HIV-1.

The other critical phase of the viral cycle of the virus is the proteolysis of the protein polypeptide precursors and mature enzymes. All the protease inhibitors (PIs) that are currently commercially available such as anti HIV/AIDS are non-hydrolysable peptidomimetics in which the peptide bond is replaced by an isoster (statin, norstatin, hydroxyethylene).

The enfuviride (T-20) is a synthetic peptide and is the first compound that is active as an entry inhibitor to have been introduced into therapy. The enfuviride stops the entry of the virus into the host cell by interfering with the fusion process. This single-action mechanism makes T-20 active against forms of HIV-1 that are resistant to the aforementioned three other classes of antiretroviral drugs (NRTIs, NNRTIs and PIs). On the basis of the clinical data, it can be concluded that long-term suppression of the virus by T-20 is possible if the latter is used in combination with the other antiretroviral drugs.

The non nucleoside RT inhibitors are one of the most important classes of compounds for the treatment of HIV-1 infections. Nevertheless, the development of more active compounds that are more resistant to mutations and are less toxic is required.

The authors of the present invention have synthesised compounds with a general structure **1** and **2** (Botta et al. J. Comb. Chem. 2005, 7, 117):
R₁= R₂= ethyl, propyl, butyl, propargyl
R₁= methyl and R₂= benzyl
Ar = phenyl, p-fluorophenyl
m- trifluoromethyl, o-methoxy 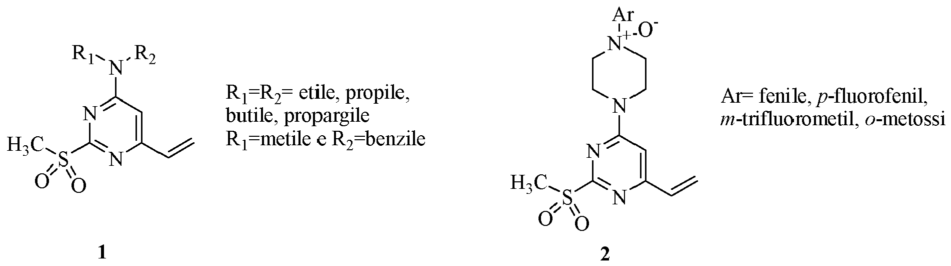 that comprises the compound MB3B.

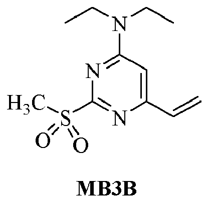

In the present invention, other compounds have been synthesised that show an inhibiting activity of the RT of HIV-1 that is greater than MB3B and a different action mechanism. The interest in new synthesised compounds has developed above all through the fact that one of the different derivatives obtained has an inhibiting activity in relation to the RT of HIV-1 that is 3 orders of magnitude greater than that of MB3B, opening up a new branch of research into the functionalisation of this latest derivative.

### DISCLOSURE OF THE INVENTION

The object of the present invention is a compound of general formula I or II 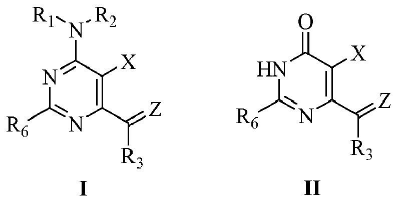 in which:
R₁ and R₂ represent independently H, ethyl, methyl, propyl, butyl, pentyl, propargyl and allyl;
X represents H, I, Cl, Br, methyl, propyl or substituted alkyl, aryl or aralkyl substituted groups;
Z represents CH₂, O, NH
R₃ represents H or an aryl with the formula: 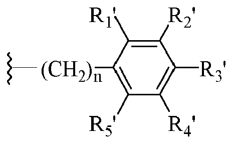 in which R₁', R₂', R₃', R₄', R₅' are independently H, C₁₋₆alkyl, C₂-₆alkenyl, C₂₋₆alkynyl, aryl substituted groups, halo, haloalkyl (in particular CF₃), OCH₃, NO₂, CN, CONH₂, CONH-C₁-₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is -H, or alkyl, aryl or aralkyl substituted groups, n is comprised between 0 and 4;
R₆ represents Y-R₇, 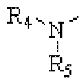 or inn 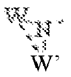 n which:
Y = S, SO or SO₂,
R₇ represents methyl, ethyl, propyl, butyl, pentyl, cyclopentyl, cyclohexyl, or aryl substituted groups with the formula: 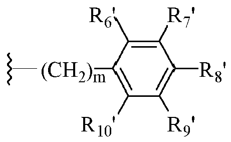 in which R₆', R₇', R₈', R₉', R₁₀' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁-₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups , m is comprised between 0 and 4;
R₇ also represents substituted cinnamoils with the formula: 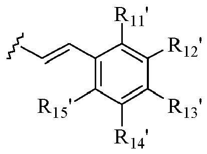 in which R₁₁' R₁₂', R₁₃', R₁₄', R₁₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups;
R₇ also represents substituted systems with the formula: 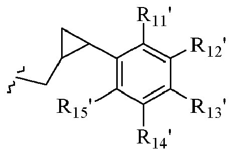 in which R₁₁', R₁₂', R₁₃', R₁₄', R₁₅' are independently H, C₁₋₆alkyl, C₂₋₆alkenyl, C₂-₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups;
R₇ also represents substituted systems with the formula: 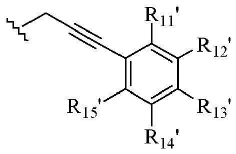 in which R₁₁', R₁₂', R₁₃', R₁₄', R₁₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁-₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups;
R₇ also represents substituted systems with the formula: 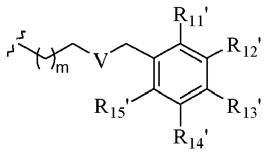 in which V is N, S, 0 and R₁₁', R₁₂', R₁₃', R₁₄', R₁₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups, m is comprised between 0 and 4;
R₄ and R₅ represent independently H, methyl, ethyl, benzyl, cyclopentyl, allyl, propargyl, pentyl, aryl substituted groups with the formula: 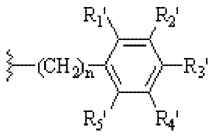 in which R₁', R₂', R₃', R₄', R₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁-₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups, n is comprised between 0 and 4; R₄ and R₅ represent independently H, methyl, ethyl, benzyl, cyclopentyl, allyl, propargyl, pentyl, aryl substituted groups with the formula 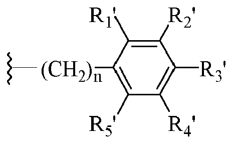 in which R₁', R₂', R₃', R₄', R₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁-₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups, n is comprised between 0 and 4;
W is C₁₋₆ alkyl or amine;
   -- represents a single or double bond;
W' is C₁₋₆ alkyl substituted groups, having optionally one or more heteroatoms being, but not only, O, N, or S;
   or a pharmaceutically acceptable salt, prodrug or tautomer thereof;
   in which the formula I compounds with the following substitutes are not included:
   R₁=R₂= ethyl, propyl, butyl or propargyl, X=H, Z=CH₂, R₃=H, R₆=Y-R₇ in which Y=S or SO₂ and R₇=methyl, and
   R₁= methyl, R₂= benzyl, X=H, Z=CH₂, R₃=H, R₆=Y-R₇ in which Y=S or SO₂ and R₇=methyl; The compound preferably has the formula I in which R₁=R₂=CH₃, X=H, Z=CH₂, R₃=H, R₆=Y-R₇ in which Y=SO₂, R₇=CH₃.

The compound preferably has the formula I in which R₁=R₂=CH₃, X=I, Z=CH₂, R₃=H, R₆= Y-R₇ in which Y=SO₂, R₄=CH₃.

The compound preferably has the formula I in which R₁=R₂=CH₂-CH₃, X=I, Z=CH₂, R₃=H, R₆=Y-R₇ in which Y=SO₂, R₇=CH₃;

The compound preferably has the formula I in which R₁=R₂=CH₂-CH₃, X=H, Z=CH₂, R₃=H, R₆=Y-R₇ in which Y=SO₂,R₇ =p-methoxybenzyl;

The compound preferably has the formula I in which R₁, R₂, R₃ and X=H, Z=CH₂, R₆=Y-R₇ in which Y=SO₂, R₇=CH₃.

The compound preferably has the formula I in which R₁=R₂=H, X=CH₃, Z=O, R₃=*p-*fluorophenyl, R₆=Y-R₇ in which Y=S, R₇=*p*-methoxybenzyl.

The compound preferably has the formula I in which R₁=R₂=H, X=CH₃, Z=O, R₃=*or-*dichlorophenyl, R₆=Y-R₇ in which Y=S, R₇=*p*-methoxybenzyl.

The compound preferably has the formula I in which R₁=R₂=H, X=CH₃, Z=O, R₃=*or-*difluorophenyl, R₆=Y-R₇ in which Y=S, R₇=*p*-methoxybenzyl.

The compound preferably has the formula I in which R₁=R₂=CH₂-CH₃, X=H, Z=CH₂, R₃=H, R₆=Y-R₇ in which Y=S, R₇ =*p*-methoxybenzyl.

The compound preferably has the formula I in which R₁=R₂=CH₃, X=H, Z=CH₂, R₃=H, R₆=Y-R₇ in which Y=S, R₇ =*p*-methoxybenzyl.

The compound preferably has the formula I in which R₁=R₂ =CH₃, X=H, Z=CH₂, R₃=H, R₆ = 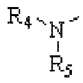 in which R₄=H, R₅= benzyl.

The compound preferably has the formula I in which R₁=R₂ =CH₃, X=H, Z=CH₂, R₃=H, R₆ = 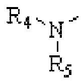 in which R₄ = methyl, R₅ = ethyl.

The compound preferably has the formula I in which R₁=R₂=CH₃, X=H, Z=CH₂, R₃=H, R₆ = 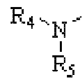 in which R₄=H, R₅ = *p*-cyanophenyl.

The compound preferably has the formula II in which:
X=H, Z=CH₂, R₃=H, R₆ = Y-R₇ in which Y=SO₂, R₇=CH₃.

The compound preferably has the formula II in which X=CH₃, Z=O, R₃ = *p*-fluorophenyl, R₆=Y-R₇ in which Y=S, R₇=*p*-methoxybenzyl.

The compound preferably has the formula II in which X=CH₃, Z=O, R₃ = *or*-dichlorophenyl, R₆=Y-R₇ in which Y=S, R₇=*p*-methoxybenzyl.

The compound preferably has the formula II in which X=CH₃, Z =O, R₃ = *or*-difluorophenyl, R₆=Y-R₇ in which Y=S, R₇=*p*-methoxybenzyl.

The compound preferably has the formula II in which X=H, Z=CH₂, R₃ =H, R₆ = 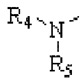 in which R₄=H and R₅ = benzyl.

The compound preferably has the formula II in which X=H, Z=CH₂, R₃=H, R₆ = 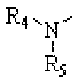 in which R₄=methyl and R₅ = ethyl.

The compound preferably has the formula II in which X=H, Z=CH₂, R₃=H, R₆ = 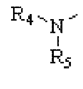 in which R₄=H and R₅=*p*-cyanophenyl.

Still another object of the present invention is a compound of formula III or IV, 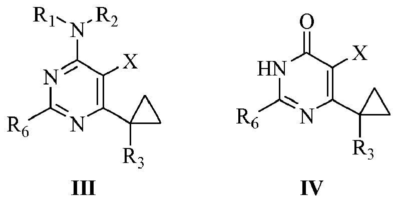 in which:
R₁ and R₂ represent independently H, ethyl, methyl, propyl, butyl, pentyl, propargyl and allyl;
X represents H, I, Cl, Br, methyl, propyl or alkyl groups, aryl or aralkyl substituted groups;
R₃ represents H or an aryl with the formula: 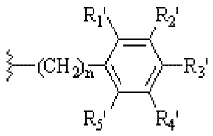 in which R₁', R₂', R₃', R₄', R₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁-₆alkyl, CON(C₁₋₆alkyl)₂, NH₂, NH-C₁₋₆alkyl, N(C₁₋₆alkyl)₂, NHC(O)alkyl, NHSO₂₋C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups, n is comprised between 0 and 4;
R₆ represents Y-R₇, 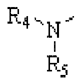 or 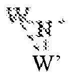 in which:
Y = S, SO or SO₂,
R₇ represents methyl, ethyl, propyl, butyl, pentyl, cyclopentyl, cyclohexyl, or aryl substituted groups of formula: 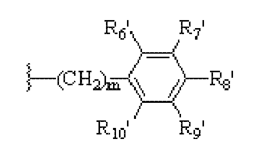 in which R₆', R₇', R₈', R₉', R₁₀' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁-₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups, m is comprised between 0 and 4;
R₄ and R₅ represent independently H, methyl, ethyl, benzyl, cyclopentyl, allyl, propargyl, pentyl, aryl substituted groups with the formula: 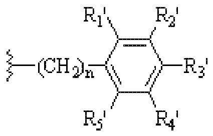 in which R₁', R₂', R₃', R₄', R₅' are independently H, C₁₋₆alkyl, C₂₋₆alkenyl, C₂₋₆alkenyl, aryl substituted groups, halo, haloalkyl (in particular CF₃), OCH₃, NO₂, CN, CONH₂, CONH-C₁-₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl groups, aryl or aralkyl substituted groups, n is comprised between 0 and 4;
W is C₁₋₆ alkyl or amine;
   -- represents a single or double bond;
W' is C₁₋₆ alkyl substituted groups, having optionally one or more heteroatoms being, but not only, O, N, or S;
   or a pharmaceutically acceptable salt, prodrug or tautomer thereof.

Another object of the present invention is a compound of formula V: 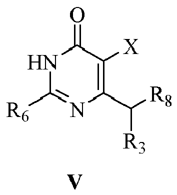 in which:
X represents H, I, Cl, Br, methyl, propyl or alkyl groups, aryl or aralkyl substituted groups;
R₃ represents H or an aryl with the formula: 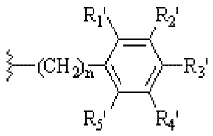 in which R₁', R₂', R₃', R₄', R₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups, n is comprised between 0 and 4;
R₆ represents Y-R₇, 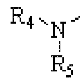 or 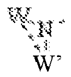 in which:
Y = N, S, SO or SO₂,
R₇ represents substituted cinnamoils with the formula: 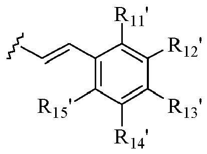 in which R₁₁', R₁₂', R₁₃', R₁₄', R₁₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C_{1- 6}alkyl, SO₂NH₂, SO₂NHC₁₋₆alkyl, SO₂N(C₁₋₆alkyl)₂, OZ' or SZ' where Z' is H, or alkyl, aryl or aralkyl substituted groups;
R₇ also represents substituted systems with the formula: 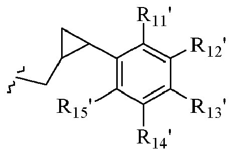 in which R₁₁', R₁₂', R₁₃', R₁₄', R₁₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups;
R₇ also represents substituted systems with the formula: 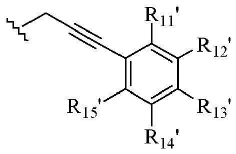 in which R₁₁', R₁₂', R₁₃', R₁₄', R₁₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups;
R₇ also represents substituted systems with the formula: 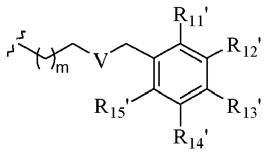 in which V is N, S, 0 and R₁₁', R₁₂', R₁₃', R₁₄', R₁₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₁-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups, m is comprised between 0 and 4;
R₄ and R₅ represent independently H, methyl, ethyl, benzyl, cyclopentyl, allyl, propargyl, pentyl, aryl substituted groups with the formula: 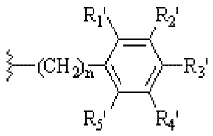 in which R₁', R₂', R₃', R₄', R₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁-₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups, n is comprised between 0 and 4;
W is C₁₋₆ alkyl or amine;
   -- represents a single or double bond;
W' is C₁₋₆ alkyl substituted groups, having optionally one or more heteroatoms including, but not only, O, N, or S;
R₈ represents methyl, =O, =CH₂;
or a pharmaceutically acceptable salt, prodrug or tautomer thereof.

An object of the present invention is a pharmaceutical composition comprising a pharmaceutically effective and acceptable quantity of a compound of general formula I, II, III, IV or V. The composition preferably comprises at least another compound having an anti-HIV activity.

Still another object of the present invention is the use of the compound of general formula I, II, III, IV or V for preparing a medicament with antiviral activity. The activity is in particular anti-HIV.

The object of the present invention is a method for the preparation of a compound of general formula I or II comprising the phases shown in diagram 1 or 2 or 3 or 4.

Another object of the present invention is method for the preparation of a compound of general formula III or IV comprising the phases shown in diagram 5.

Another object of the present invention is method for the preparation of a compound of general formula V comprising the phases shown in diagram 6, 7 or 8.

It is an object of the invention a compound of formula V: 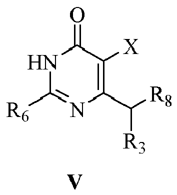 in which:
X represents H, I, Cl, Br, methyl, ethyl, propyl or alkyl, aryl or aralkyl substituted groups;
R₃ represents an aryl with the formula: 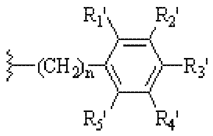 in which R₁', R₂', R₃', R₄', R₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁-₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups, n is comprised between 0 and 4;
R₆ represents Y-R₇, 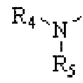 in which:
Y = S, SO or SO₂,
R₇ represents methyl, ethyl, propyl, butyl, pentyl, cyclopentyl, cyclohexyl, aryl substituted groups with the formula: 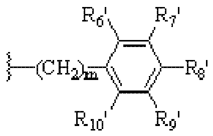 in which R₆', R₇', R₈', R₉', R₁₀' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁-₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups, m is comprised between 0 and 4;
R₇ also represents substituted cinnamoils with the formula: 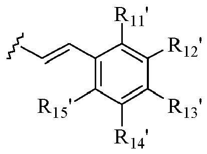 in which R₁₁', R₁₂', R₁₃', R₁₄', R₁₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₁-C_{1- 6}alkyl, SO₂NH₂, SO₂NHC₁₋₆alkyl, SO₂N(C₁₋₆alkyl)₂, OZ' or SZ' where Z' is H, or alkyl, aryl or aralkyl substituted groups;
R₇ also represents substituted systems with the formula: 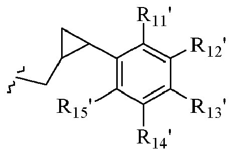 in which R₁₁', R₁₂', R₁₃', R₁₄', R₁₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups;
R₇ also represents substituted systems with the formula: 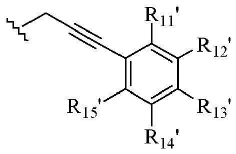 in which R₁₁', R₁₂', R₁₃', R₁₄', R₁₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups;
R₇ also represents substituted systems with the formula: 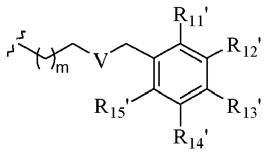 in which V is N, S, O and R₁₁', R₁₂', R₁₃', R₁₄', R₁₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups, m is comprised between 0 and 4;
R₄ and R₅ represent independently H, methyl, ethyl, benzyl, cyclopentyl, allyl, propargyl, pentyl, aryl substituted groups with the formula: 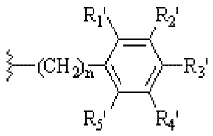 in which R₁', R₂', R₃', R₄', R₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁-₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₁NHC₁₋₆alkyl, SO₂N(C₁₋₆alkyl)₂, OZ' or SZ' where Z' is H, or alkyl, aryl or aralkyl substituted groups, n is comprised between 0 and 4;
R₈ represents H or methyl;
   or a pharmaceutically acceptable salt, prodrug, stereoisomers or tautomer thereof, wherein if X = ethyl,
-R₃ = 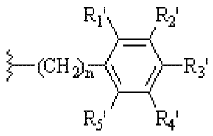 in which n = 0, R_{1'} = R_{5'}= Cl or F, R_{2'}=R_{3'}=R_{4'}= H, R₆ = Y-R₇ in which Y = S and R₇ is a substituted cinnamoils with the formula: 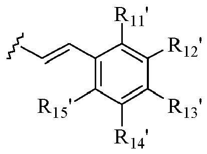 in which R₁₁', R₁₂' , R₁₄', R₁₅' = H, and R₁₃' = CN or OCH₃ or NO₂ and R₈ = H; wherein if R₈ = H
- X = methyl, R₃ represents an aryl with the formula: 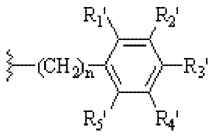 in which n = 0, R_{1'} = R_{5'}= Cl, R_{2'}=R_{3'}=R_{4'}= H, R₆ = Y-R₇ in which Y = S and R₇ is a substituted cinnamoils with the formula: 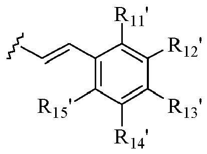 in which R₁₁', R₁₂' , R₁₄', R₁₅' = H, and R₁₃' = OCH₃, NO₂ or N(CH₃)₂; or
- X = methyl, R₃ represents an aryl with the formula: 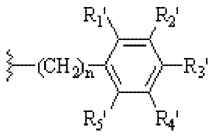 in which n = 0, R_{1'} = R_{5'}= F, R_{2'}=R_{3'}=R_{4'}= H, R₆ = Y-R₇ in which Y = S and R₇ is a substituted cinnamoils with the formula: 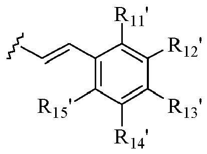 in which R₁₁', R₁₂', R₁₄', R₁₅' = H, and R₁₃' = OCH₃ or NO₂; or
- X = methyl, R₃ represents an aryl with the formula: 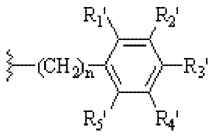 in which n = 0, R_{1'} = R_{5'}= F, R_{2'}=R_{3'}=R_{4'}= H, R₆ = Y-R₇ in which Y = S and R₇ is a substituted system with the formula: 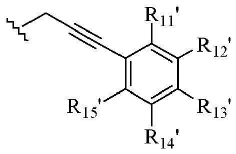 in which R_{11'}, R_{12'}, R_{14'}, R_{15'} = H and R₁₃'= NO₂; or
- X = methyl, R₃ represents an aryl with the formula: 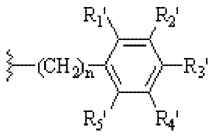 in which n = 0, R_{1'} = R_{5'}= Cl, R_{2'}=R_{3'}=R_{4'}= H, R₆ = Y-R₇ in which Y = S and R₇ is a substituted system with the formula: 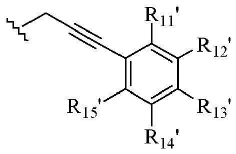 in which R₁₁', R₁₂', R_{14'}, R_{15'} = H and R_{13'}= OCH₃; or
- X = methyl, R₃ represents an aryl with the formula: 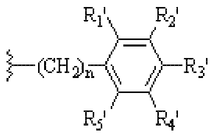 in which n = 0, R_{1'} = R_{5'} = Cl, R₂'=R₃'=R₄'= H, R₆ = Y-R₇ in which Y = S and R₇ is a substituted system with the formula: 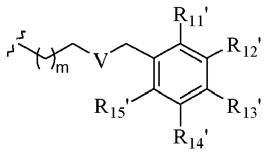 in which m is 1, V is O and R₁₁', R₁₂', R₁₄', R₁₅' are H and R₁₃'= OCH₃.

It is an object of the invention a compound of formula V as defined above in which:
X represents H, I, Cl, Br, methyl, ethyl, propyl or alkyl, aryl or aralkyl substituted groups; R₃ represents an aryl with the formula: 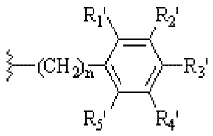 in which R₁', R₂', R₃', R₄', R₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁_ ₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups, n is comprised between 0 and 4;
R₆ represents Y-R₇, in which:
   Y = S, SO or SO₂,
R₇ represents substituted cinnamoils with the formula: 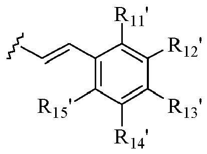 in which R₁₁', R₁₂', R₁₃', R₁₄', R₁₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups;
R₇ also represents substituted systems with the formula: 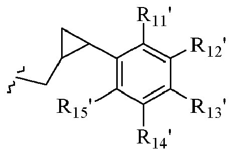 in which R₁₁', R₁₂', R₁₃', R₁₄', R₁₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups;
R₇ also represents substituted systems with the formula: 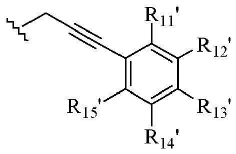 in which R₁₁', R₁₂', R₁₃', R₁₄', R₁₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups;
R₇ also represents substituted systems with the formula: 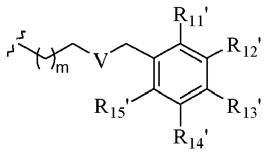 in which V is N, S, O and R₁₁', R₁₂', R₁₃', R₁₄', R₁₅' are independently H, C₁₋₆alkyl, C₂₋₆ alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups, m is comprised between 0 and 4;
R₈ represents H or methyl;
   or a pharmaceutically acceptable salt, prodrug, stereoisomers or tautomer thereof, wherein if X = ethyl, -R₃ = 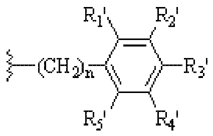 in which n = 0, R₁' = R₅' = Cl or F, R_{2'}=R_{3'}=R_{4'}= H, R₆ = Y-R₇ in which Y = S and R₇ is a substituted cinnamoils with the formula: 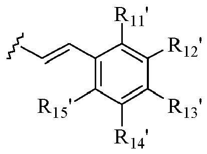 in which R₁₁', R₁₂' , R₁₄', R₁₅' = H, and R₁₃' = CN or OCH₃ or NO₂ and R₈ = H; wherein if R₈ = H
- X = methyl, R₃ represents an aryl with the formula: 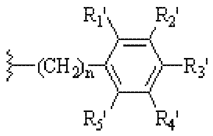 in which n = 0, R_{1'} = R_{5'} = Cl, R_{2'}=R_{3'}=R_{4'} = H, R₆ = Y-R₇ in which Y = S and R₇ is a substituted cinnamoils with the formula: 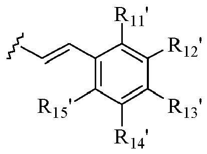 in which R₁₁', R₁₂' , R₁₄', R₁₅' = H, and R₁₃' = OCH₃, NO₂ or N(CH₃)₂; or
- X = methyl, R₃ represents an aryl with the formula: 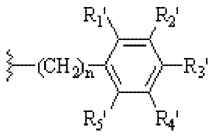 in which n = 0, R_{1'} = R_{5'} = F, R_{2'}=R_{3'}=R_{4'}= H, R₆ = Y-R₇ in which Y = S and R₇ is a substituted cinnamoils with the formula: 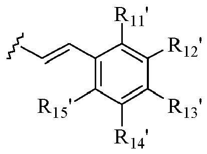 in which R₁₁', R₁₂' , R₁₄', R₁₅' = H, and R₁₃' = OCH₃ or NO₂; or
- X = methyl, R₃ represents an aryl with the formula: 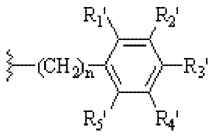 in which n = 0, R_{1'} = R_{5'} = F, R_{2'}=R_{3'}=R_{4'}= H, R₆ = Y-R₇ in which Y = S and R₇ is a substituted system with the formula: 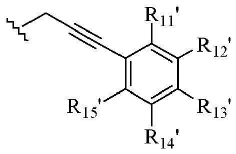 in which R₁₁', R₁₂', R_{14'} , R₁₅' = H and R₁₃'= NO₂; or
- X = methyl, R₃ represents an aryl with the formula: 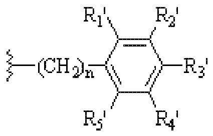 in which n = 0, R_{1'} = R_{5'} = Cl, R_{2'}=R_{3'}=R_{4'}= H, R₆ = Y-R₇ in which Y = S and R₇ is a substituted system with the formula: 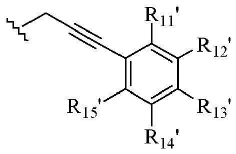 in which R₁₁', R₁₂', R_{14'}, R₁₅' = H and R₁₃'= OCH₃; or
- X = methyl, R₃ represents an aryl with the formula: 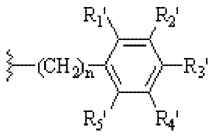 in which n = 0, R₁' = R₅' = Cl, R₂'=R₃'=R₄'= H, R₆ = Y-R₇ in which Y = S and R₇ is a substituted system with the formula: 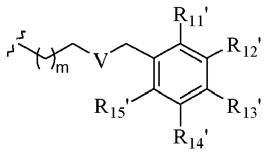 in which m is 1, V is O and R₁₁', R₁₂', R₁₄', R₁₅' are H and R₁₃'= OCH₃.

It is an object of the invention the compound as defined above for medical use. Preferably, the compound is for use as an anti-viral agent. More preferably, the anti-viral activity is anti-HIV. It is a further object of the invention a pharmaceutical composition comprising a pharmaceutically effective and acceptable quantity of the compound of formula V as defined above and suitable dilutants.

Preferably, the pharmaceutical composition further comprises at least another compound having an anti-HIV activity.

It is an object of the invention a method for the preparation of a compound of general formula V as defned above, comprising the phases shown in diagrams 6, 7 or 8.

The present invention will now be disclosed by way of non-limiting example with particular reference to the following figures:
Figure 1. Titrations of **8a** in a reverse transcription reaction *in vitro* with recombinant RT and in the presence of different concentrations of nucleotide substrate (in this case dTTP); ID50: concentration of inhibitor required to inhibit 50% of enzyme activity; pmol: Vmax/Km
Figure 2. A) Titrations of nucleotide substrate (dTTP) in a reverse transcription reaction *in vitro* with recombinant RT and in the presence of different concentrations of **8a**. B) Kapp: concentration of substrate required for having 50% of maximum reaction speed; pmol: Vmax/Km
Figure 3. Titrations of **8a** in an *in vitro* reverse transcription reaction *in vitro* with recombinant RT and in the presence of different concentrations of nucleic acid substrate (in this case poly(rA)/oligo(dT)).

### STRUCTURE AND SYNTHESIS OF COMPOUNDS

The general structure compounds **I** and **II** in which R₄ = CH₃, R₃ = H, Z = CH₂ and R₆=Y-R₇ in which Y=S, SO, SO₂, R₇=CH₃ (**6, 7, 8, 9a, 11a**) are synthesised with the procedure shown in Diagram 1.

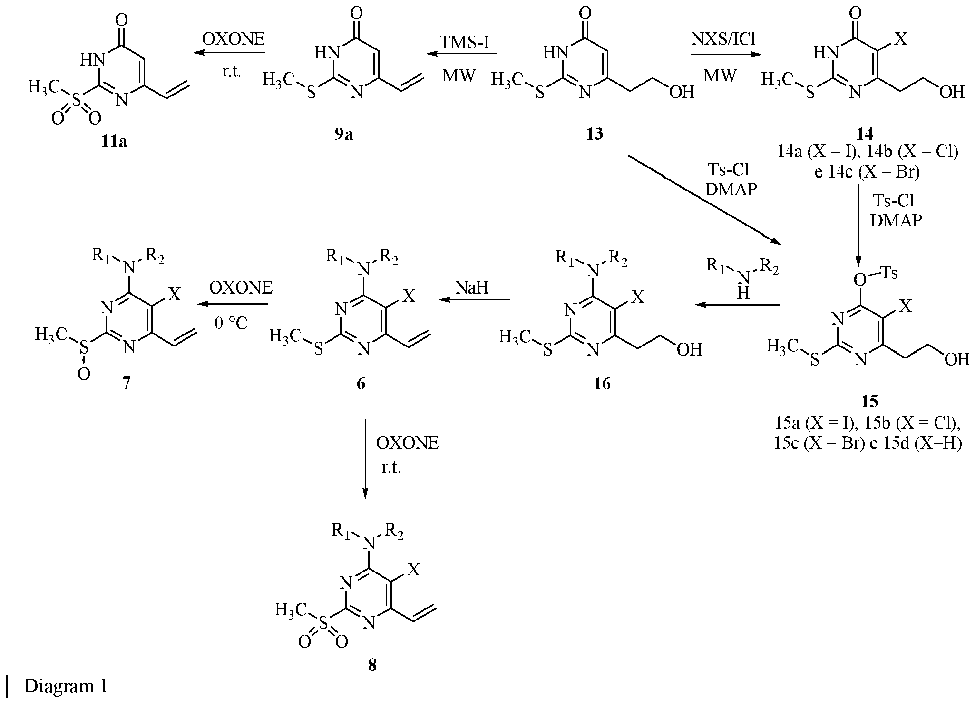

The general structure compound **13** is known in the literature and synthesised according to previously reported methods (Botta, M.; Occhionero, F.; Saladino, R.; Crestini, C.; Nicoletti, R. Tetrahedron Lett. 1997, 38, 8249-8252). The synthesis method for obtaining the compounds **8** with X = H and **11a** is shown in Botta et al. J. Comb. Chem. 2005, 7, 117.

For the compounds in which X = I, Cl, Br a general procedure was used that was previously devised in our laboratories for the halogenation of functionalised pyrimidinones (Paolini, L.; Petricci, E.; Corelli, F.; Botta, M Synthesis 2003, 1039-1044.).

In order to obtain the general structure compounds **I** in which R₆=Y-R₇ with Y=SO₂ and R₇ other than CH₃ the procedure shown in Diagram 2 was used.

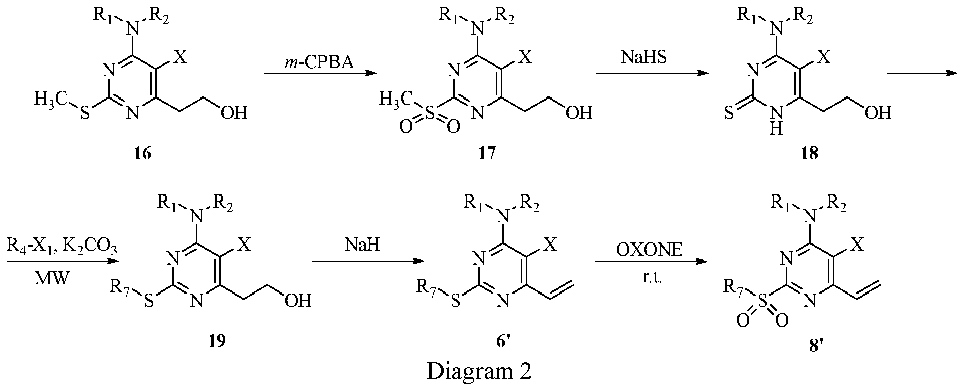

The procedure for synthesising the product **18** was developed after several attempts starting with the product **16** through sulphonic oxidation **17** and subsequent substitution with NaHS; other commonly used reagents for obtaining products with a correlated structure have not given satisfactory results in the case of this type of derivative. Functionalisation of the product **18** thus continues for treatment with a suitable halogen derivative in a microwave oven in the presence of K₂CO₃, subsequent dehydration (**6'**) and oxidation of the S with OXONE^{®} at 0°C to obtain selectively sulphoxide (Y=SO) or at ambient temperature to isolate the sulphone (Y=SO₂)

In order to obtain the derivatives with a general formula **II** in which Z=O, diagram 3 has been followed.

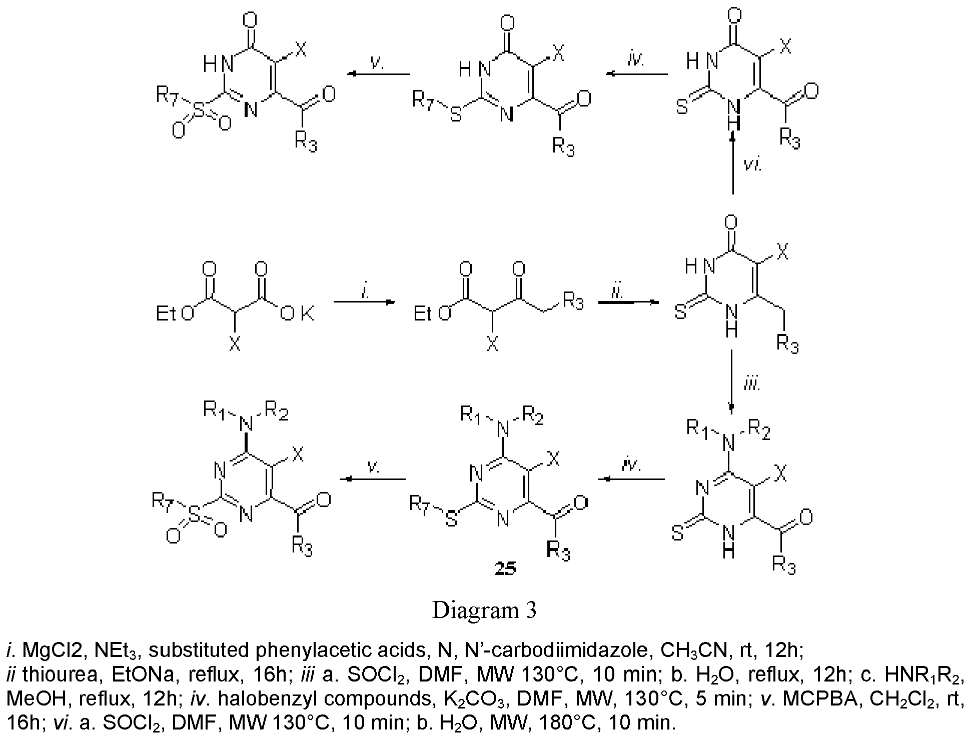

In order to obtain products with a general structure **I** and **II** with R₆ = 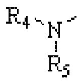 or 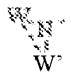 another synthetic strategy was used that replaces the methyl group in position two with a suitable amine through a "one-pot" reaction in which the S atom is first oxidised with *m-*chloroperbenzoic acid and is then substituted by a suitable amine (Diagram 4).

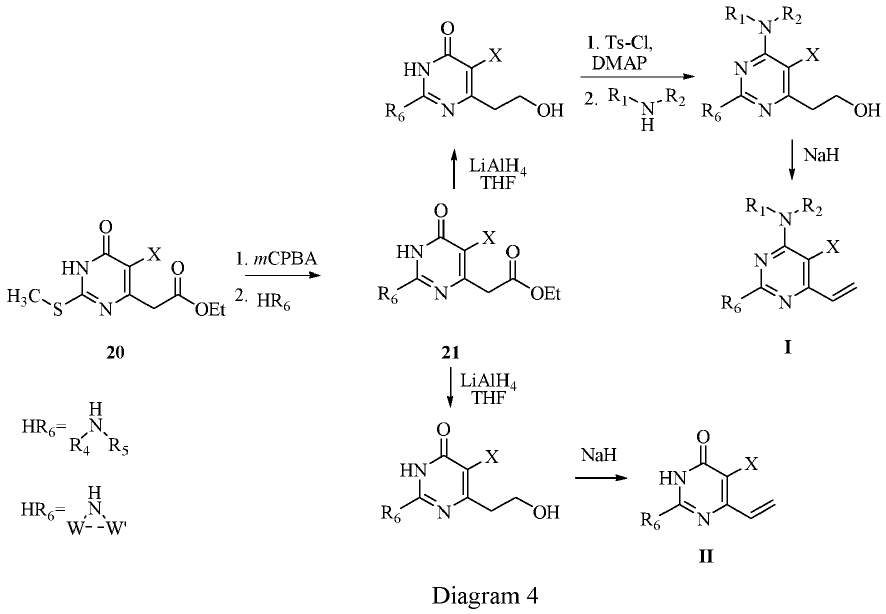

The proposed procedure is the only one that provided satisfactory results for our purposes. Once the replaced product **21** has been obtained the ester is reduced to alcohol and procedure is conducted in the same conditions for the synthesis of the compounds **15, 16, 6** and **9a,** respectively.

The procedure was applied to compounds with Z = CH₂, R₃ = H but those skilled in the art will be able to apply it to compounds with different Z and R₃. In fact, in the case of the compounds with Z = CH₂, R₃ = H is the most complex chemically both as a synthesis procedure and as functionalisation in the different phases.

In order to obtain products with a general structure **III** in which R₆=Y-R₇ the synthetic procedure shown in diagram 5 was followed:

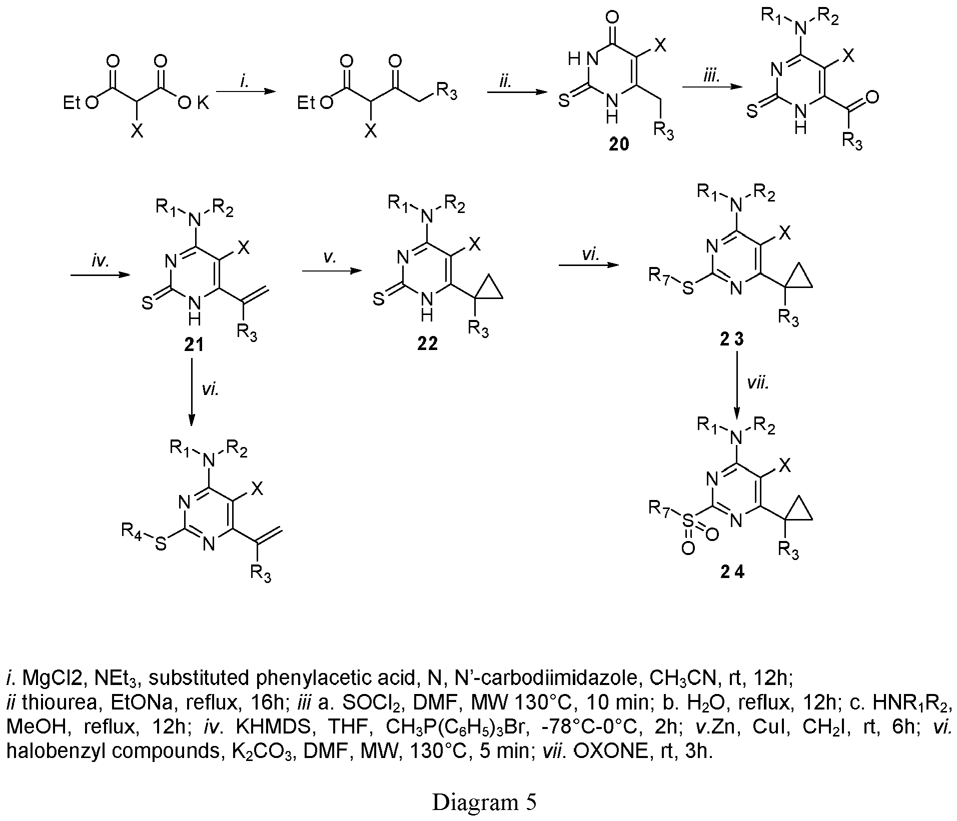

The suitable cycled product **20,** obtained with the classic procedures found in the literature for the synthesis of similar compounds (Box, V. G. S.; Marinovich, N.; Yiannikouros, G. P. Heterocycles 1991, 2, 245-251.), was subjected to complete chloridisation through microwave treatment with SOCl₂ in DMF and is then transformed into corresponding carbonyl derivative for H₂O treatment at reflux over 12 hours. The obtained derivative was then treated with a suitable amine in order to obtain the functionalisation in C4 and was then subj ected to a Wittig reaction, supplying the vinil derivative **21** with good yields. Through cyclopropanation of the double bond the compound **22** was obtained that was then functionalised in C2 to supply the derivatives **III** for treatment with suitable benzyl halides substituted in DMF, in the presence of K₂CO₃ in a microwave oven for 5 minutes (Petricci, E.; Mugnaini, C.; Radi, M.; Corelli, F.; Botta, M. J. Org. Chem. 2004, 69, 7880-7887.). The corresponding sulphoxides and sulphides of the derivative **23** were obtained through treatment of the latter with OXONE^{®} at 0°C and ambient temperature respectively.

For the synthesis of the general formula derivatives **III** with R₆ = 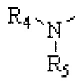 or

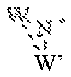

The derivative **24** was treated at reflux with suitable amines.

The general structure compounds **V** are synthesised with the procedure indicated in Diagram 6, 7 or 8.

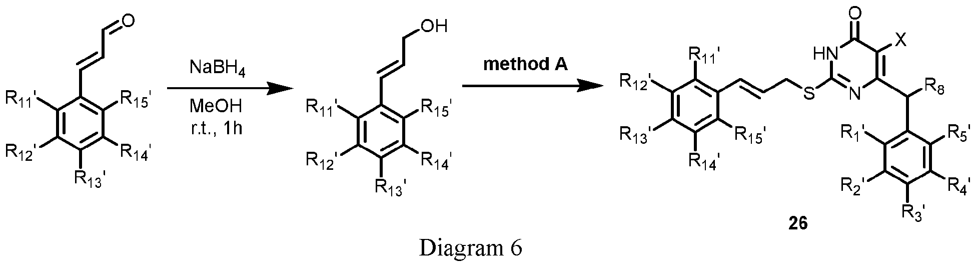

| **Comp.** | **R13'** | **X** | **R₈** | **R₁' and R₅'** |
|---|---|---|---|---|
| 26a | OCH₃ | CH₃ | H | Cl |
| 26b | NO₂ | CH₃ | H | Cl |
| 26c | CN | C₂H₅ | H | Cl |
| 26d | N(CH₃)₂ | CH₃ | H | Cl |
| 26e | OCH₃ | C₂H₅ | H | Cl |
| 26f | NO₂ | C₂H₅ | H | Cl |
| 26g | OCH₃ | CH₃ | CH₃ | Cl |
| 26h | NO₂ | CH₃ | CH₃ | Cl |
| 26i | NO₂ | CH₃ | H | F |
| 26l | OCH₃ | CH₃ | H | F |
| 26m | NO₂ | CH₃ | CH₃ | F |
| 26n | OCH₃ | CH₃ | CH₃ | F |
| 26o | CN | CH₃ | CH₃ | F |
| 26p | OCH₃ | C₂H₅ | CH₃ | F |
| 26q | NO₂ | C₂H₅ | CH₃ | F |
| 26r | CN | C₂H₅ | CH₃ | F |

R₁₁', R₁₂', R₁₄', R₁₅', R₂', R₃', R₄' = H

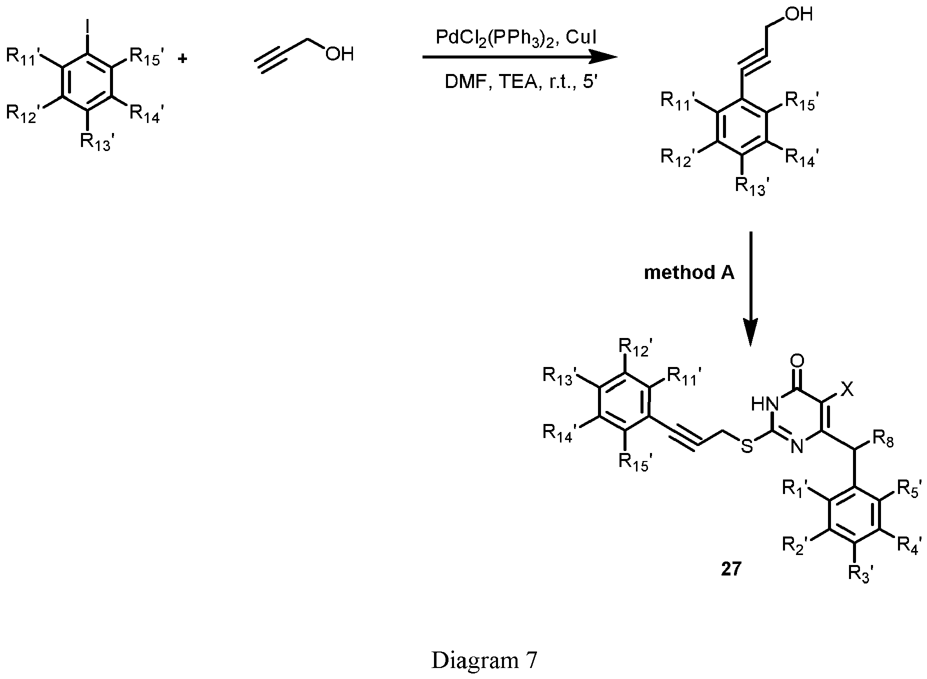

| **Comp.** | **R₁₄'** | **X** | **R₈** | **R₁' and R₅'** |
|---|---|---|---|---|
| 27a | NO₂ | CH₃ | H | F |
| 27b | OCH₃ | CH₃ | CH₃ | Cl |
| 27c | OCH₃ | CH₃ | H | Cl |

R₁₁', R₁₂', R₁₄', R₁₅', R₂', R₃', R₄' = H

### Method A:

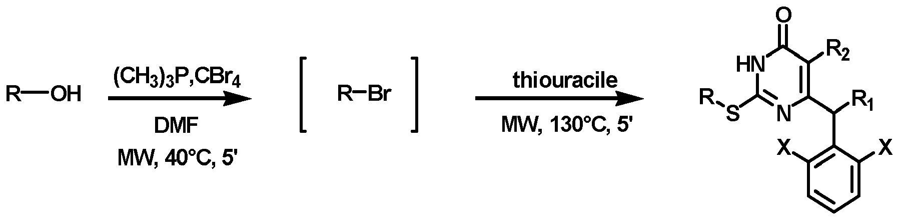

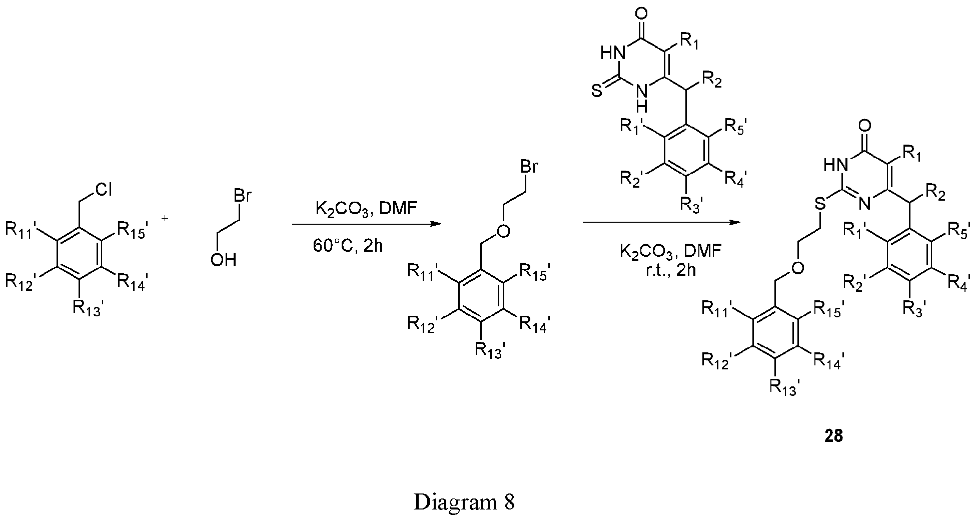

### EXAMPLES

### EXAMPLE 1

Preparation of the compound **14a:** 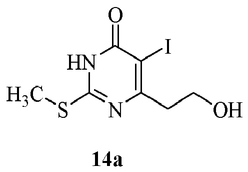
a) react the product **13** (5.4 mmol) with ICl (10.8 mmol) in anhydrous DMF (25 mL) at MW (power max = 200W) at 50°C for 15 minutes;
b) the DMF is evaporated in N₂ and the obtained product is purified through chromatography on a silica gel column using a 95/5 CH₂Cl₂/MeOH mixture as an eluent (yield 60%).
¹H-NMR (CD₃OD): δ (ppm) 3.93-3.87 (t, *J*=6.67, 2H); 3.06-2.99 (t, *J*=6.67, 2H); 2.54 (s, 1H).
MS: *m*/*z* 313 [M+1]⁺; 335 [M+Na]⁺; 646 [2M+Na]⁺.

### EXAMPLE 2

Preparation of the compound **14b**: 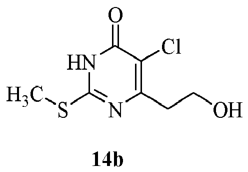
c) react the product **13** (5.4 mmol) with NCS (10.8 mmol) in anhydrous DMF (25 mL) at MW (power max = 200W) at 50°C for 15 minutes;
d) the DMF is evaporated in N₂ and the obtained product is purified on a silica gel column by chromatography using a 9/1 mixture of CH₂Cl₂/MeOH as an eluent (yield 54%).
¹H-NMR (CD₃OD): δ (ppm) 3.94-3.89 (t, *J*=6.49, 2H); 3.02-2.95 (t, *J*=6.49, 2H); 2.55 (s, 3H).
m.p.: 168-170 °C
MS: *m*/*z* 243 [M+Na]⁺.

### EXAMPLE 3

Preparation of the compound **14c:** 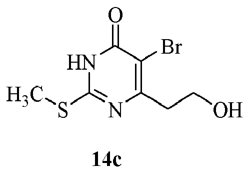
a) react the product **13** (5.4 mmol) with NBS (10.8 mmol) in anhydrous MeOH (25 mL) in the presence of DTBP (16.2 mmol) at ambient temperature for 1h;
b) the solvent is evaporated at reduced pressure and the obtained product is purified on a silica gel column by chromatography using a 9/1 mixture of CH₂Cl₂/MeOH as an eluent (yield 69%).
¹H-NMR (CD₃OD): δ (ppm) 3.92-3.87 (t, *J*=6.50, 2H); 3.03-2.97 (t, *J*=6.50, 2H); 2.55 (s, 3H).
m.p.:166-169°C
MS: *m*/*z* 266 [M+1]⁺.

### EXAMPLE 4

Preparation of the compounds **15(a-d)**: 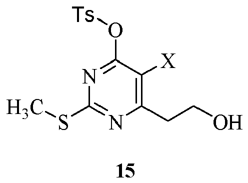 in which for 15a (X = I), 15b (X = Cl) and 15c (X = Br) and 15d (X =H)
a) react the product **14 a-c** (**13** if X = H) (5.4 mmol) with *p*-toluenesulphonyl chloride (6.5 mmol) and DMAP (8.1 mmol) in anhydrous DMF (20 mL) at MW (power max = 200W) at 45 °C for 6 minutes;
b) dilute with CH₂Cl₂ (20 mL), wash the mixture with a water solution of HCl 2N then with a saturate solution of NaCl;
c) dry the organic phase on anhydrous Na₂SO₄, filter and evaporate the solvent at reduced pressure;
d) purify the raw reaction through chromatography on silica gel using a 95/5 CH₂Cl₂/MeOH mixture as an eluent providing the corresponding products **15a-d**.
   **15a:** X=I (yield 73%)
      ¹H-NMR (CDCl₃): δ (ppm) 7.95-7.83 (d, *J*=8.00, 2H); 7.46-7.38 (d, *J*=8.00, 2H); 3.92-3.88 (t, *J*=6.78, 2H); 2.89-2.77 (t, *J*=6.78, 2H); 2.44 (s, 3H); 2.35 (s, 3H).
      m.p.:122-124°C
      MS: *m*/*z* 466 [M+1]⁺; 488 [M+Na]⁺.
   **15b:** X=Cl (yield 70%)
      ¹H-NMR (CDCl₃): δ (ppm) 7.92-7.85 (d, *J*=8.50, 2H); 7.44-7.37 (d, *J*=8.50, 2H); 3.95-3.91 (t, *J*=6.45, 2H); 2.86-2.76 (t, *J*=6.45, 2H); 2.41 (s, 3H); 2.32 (s, 3H).
      m.p.: 94-95 °C
      MS: *m*/*z* 398 [M+Na]⁺.
   **15c:** X=Br (yield 31%)
      ¹H-NMR (CDCl₃): δ (ppm) 7.95-7.87 (d, *J*=7.97, 2H); 7.42-7.35 (d, *J*=7.97, 2H); 3.97-3.90 (t, *J*=5.70, 2H); 2.86-2.79 (t, *J*=5.70, 2H); 2.39 (s, 3H); 2.35 (s, 3H).
      MS: *m*/*z* 458 [M+1]⁺; 442 [M+Na]⁺.
   **15d:** X=H (yield 92%)
      ¹H-NNM (CDCl₃): δ (ppm) 7.77-7.73 (d, *J*=8.46, 2H); 7.22-7.18 (d, *J*=8.46, 2H); 6.46 (s, 1H); 3.81-3.75 (t, *J*=5.70, 2H); 2.77-2.71 (t, *J*=5.70, 2H); 2.29 (s, 3H); 2.19 (s, 3H).
      MS: *m*/*z* 341 [M+1]⁺; 363 [M+Na]⁺.

### EXAMPLE 5

Preparation of the compound **16:** 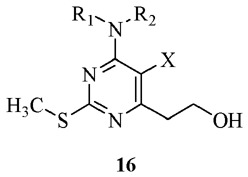 in which X = I, Cl, Br, H; R₁=R₂= ethyl, H; R₁= H and R₂= pentyl, propargyl, allyl.
a) react the product **15 a,b,c,d** (0.21 mmol) in anhydrous THF (8 mL) with the appropriate amine (0.32 mmol) at 70 °C for 12 hours;
b) bring to ambient temperature;
c) add the scavenger PS-1,3,4,6,7,8-hexahydro-2*H*- pyrimido[1,2-*a*]pyrimidino (2 equiv/mol) and subject the mixture to magnetic stirring at ambient temperature for 2 hours;
d) add the scavenger PS-isocyanate (2 equiv/mol) and subject the mixture to magnetic stirring at ambient temperature for another 2 hours;
e) filter the mixture and wash the scavengers twice with 5 mL of CH₂Cl_{2;}
f) evaporate under reduced pressure.

Analytical data on certain synthesised compounds:
**16a:** X=I, R₁=R₂= ethyl (yield 98%)
   ¹H-NMR (CDCl₃): δ (ppm) 3.96-3.90 (t, *J*=5.30, 2H); 3.58-3.47 (q, *J*=7.14, 4H); 3.00-2.95 (t, *J*=5.30, 2H); 2.44 (s, 3H); 1.23-1.16 (t, *J*=7.14, 6H).
   MS: *m*/*z* 368 [M+1]⁺; 390 [M+Na]⁺.
**16b:** X=I, R₁=H, R₂ =pentyl, (yield 49%)
   ¹H-NNM (CDCl₃): δ (ppm) 3.97-3.81 (t, *J*=5.40, 2H); 3.52-3.42 (m, 2H); 2.92-2.87 (t, *J*=5.40, 2H); 2.54 (s, 3H); 1.64-1.54(m, 2H); 1.36-1.29(m, 4H); 0.93-0.86 (m, 3H).
   m.p.: 73-75 °C
   MS: *m*/*z* 382 [M+1]⁺; 404 [M+Na]⁺.
**16c:** X=I, R₁=H, R₂=propargyl, (yield 74%)
   ¹H-NNM (CDCl₃): δ (ppm) 4.29-4.26 (m, 2H); 3.97-3.95 (m, 2H); 2.95-2.91 (t, *J*=5.00, 2H); 2.49 (s, 3H); 2.26-2.23 (m, 1H).
   m.p.: 134-136 °C
   MS: *m*/*z* 350 [M+1]⁺; 372 [M+Na]⁺.
**16d:** X=I, R₁=H, R₂=allyl, (yield 100%)
   ¹H-NMR (CDCl₃): δ (ppm) 6.02-5.81 (m, 1H); 5.27-5.13 (m, 2H); 4.16-4.10 (m, 2H); 4.10-3.96 (m, 2H); 2.96-2.90 (m, 2H); 2.52 (s, 3H).
   m.p.: 74-76 °C
   MS: *m*/*z* 352 [M+1]⁺; 374 [M+Na]⁺.
**16e:** X=Cl, R₁=H, R₂=pentyl, (yield 67%)
   ¹H-NNM (CDCl₃): δ (ppm) 3.95-3.88 (m, 2H); 3.50-3.44 (m, 2H); 2.88-2.81 (t, *J*=5.40, 2H); 2.51 (s, 3H); 1.75-1.52 (m, 2H); 1.34-1.27 (m, 4H); 0.91-0.85 (m, 3H).
   m.p.: 64-67°C
   MS: *m*/*z* 290 [M+1]⁺; 312 [M+Na]⁺.
**16f:** X=Cl, R₁=H, R₂=propargyl, (yield 37%)
   ¹H-NNM (CDCl₃): δ (ppm) 4.31-4.28 (m, 2H); 3.99-3.94 (m, 2H); 2.94-2.90 (t, *J*=5.00, 2H); 2.48 (s, 3H); 2.26-2.23 (m, 1H).
   m.p.: 114-116°C
   MS: *m*/*z* 258 [M+1]⁺; 280 [M+Na]⁺.
**16g:** X=Cl, R₁=H, R₂=allyl, (yield 79%)
   ¹H-NNM (CDCl₃): δ (ppm) 6.02-5.81 (m, 1H); 5.27-5.13 (m, 2H); 4.16-4.10 (m, 2H); 4.10-3.96 (m, 2H); 2.96-2.90 (m, 2H); 2.52 (s, 3H).
   m.p.: 74-76 °C
   MS: *m*/*z* 280 [M+Na]⁺.
**16h:** X=Br, R₁=H, R₂=pentyl, (yield 57%)
   ¹H-NNM (CDCl₃): δ (ppm) 3.95-3.84 (t, *J*=5.40, 2H); 3.52-3.42 (m, 2H); 2.93-2.89 (t, *J*=5.40, 2H); 2.54 (s, 3H); 1.64-1.54 (m, 2H); 1.36-1.29 (m, 4H); 0.93-0.86 (m, 3H).
   MS: *m*/*z* 335 [M+1]⁺; 357 [M+Na]⁺.
**16i:** X=Br, R₁=H, R₂=propargyl, (yield 65%)
   ¹H-NMR (CDCl₃): δ (ppm) 4.33-4.28 (m, 2H); 4.00-3.95 (m, 2H); 2.93-2.90 (t, *J*=5.00, 2H); 2.48 (s, 3H); 2.28-2.20 (m, 1H).
   m.p.: 113-115 °C
   MS: *m*/*z* 303 [M+1]⁺; 325 [M+Na]⁺.
**16l**: X=Br, R₁=H, R₂=allyl, (yield 62%)
   ¹H-NMR (CDCl₃): δ (ppm) 5.99-5.85 (m, 1H); 5.27-5.15 (m, 2H); 4.16-4.10 (m, 2H);
   4.10-3.96 (m, 2H); 2.94-2.92 (m, 2H); 2.48 (s, 3H).
   m.p.: 63-65 °C
   MS: *m*/*z* 328 [M+Na]⁺.

### EXAMPLE 6

Preparation of the compound **16(m-p):** 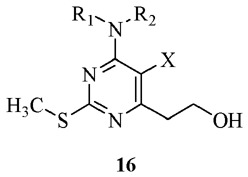 in which R₁=R₂= methyl and for **16m:** X = I, for **16n:** X = H, for **16o:** X = Cl and for **16p:** X = Br;
a) react the product **15a-d** (0.47 mmol) with dimethylamine (0.71 mmol) and K₂CO₃ (0.518 mmol) in EtOH (15 mL) at reflux for 1 hour;
b) evaporate the solution at reduced pressure and purify on a silica gel column using a 1/1 mixture of AcOEt/hexane as eluent.

Analytical data on certain synthesised compounds:
**16m:** X=I, R₁=R₂= methyl (yield 77%)
   ¹H-NNM (CDCl₃): δ (ppm) 3.98-3.85 (t, *J*=5.39, 2H); 3.11 (s, 6H); 3.09-2.95 (t, *J*=5.39, 2H); 2.48 (s, 3H).
   MS: *m*/*z* 340 [M+1]⁺.
**16n**: X=H, R₁=R₂= methyl (yield 50%)
   ¹H-NMR (CDCl₃): δ (ppm) 5.97 (s, 1H); 3.98-3.84 (t, *J*=5.40, 2H); 3.09 (s, 6H); 2.81-2.72 (t, *J*=5.40, 2H); 2.47 (s, 3H).
   MS: *m*/*z* 214 [M+1]⁺; 236 [M+Na]⁺.

### EXAMPLE 7

Preparation of the compound **6:** 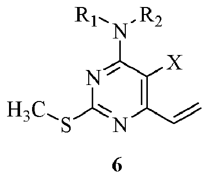 in which X = I, Cl, Br, H; R₁=R₂= methyl, ethyl, H; R₁= H and R₂= pentyl, propargyl, allyl.
a) react the compound **16** (0.1 mmol) in anhydrous dioxane(5 mL) in the presence of NaH (0.2 mmol) and is left at reflux for 3 hours;
b) bring to ambient temperature, filter and evaporate at reduced pressure.

Analytical data on certain synthesised compounds:
**6a:** X=I, R₁=R₂= methyl (yield 40%)
   ¹H-NNM (CDCl₃): δ (ppm) 7.14-7.00 (dd, *J_{cis}*=16.6, *J_{gem}*=3.35, 1H); 6.54-6.44 (?, 1H);
   5.56-5.50 (?, 1H); 3.09 (s, 6H); 2.51 (s, 3H).
   m.p.:59-61°C
   MS: *m*/*z* 322 [M+1]⁺.
**6b:** X=I, R₁=R₂= ethyl (yield 40%)
   ¹H-NNM (CDCl₃): δ (ppm) 7.15-7.01 (m, 1H); 6.51-6.41 (dd, *J_{cis}*=13.02, *J_{gem}=*2.54, 1H);
   5.53-5.47 (dd, *J_{cis}*=12.02, *J_{gem}*=2.54, 1H); 3.46-3.38 (m, 4H); 2_{.}48 (s, 3H); 1.71-1.53 (m, 4H); 0.99-0.82 (t, *J*=7.10, 6H).
   MS: *m*/*z* 378 [M+1]⁺.
**6c:** X=H, R₁=R₂= methyl (yield 45%)
   ¹H-NNM (CDCl₃): δ (ppm) 6.48-6.28 (m, 2H); 5.99 (s, 1H); 5.47-5.42 (d, *J*=10.4, 1H);
   3.05 (s, 6H); 2.49 (s, 3H).
   MS: *m*/*z* 196 [M+1]⁺.

### EXAMPLE 8

Preparation of the compound **8**: 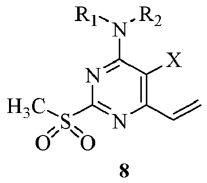 in which X = I, Cl, Br, H; R₁=R₂= methyl, ethyl, H; R₁= H and R₂= pentyl, propargyl, allyl.
a) solubilise the product **6** (1.5 mmol) in MeOH (12 mL) and add an OXONE® solution (4.5 mmol) in H₂O (12 mL). Subject to magnetic stirring at ambient temperature for 3 hours;
b) filter the mixture on cotton and evaporate at reduced pressure;
c) purify through chromatography on silica gel using a 1/1 AcOEt/hexane mixture as an eluent.

Analytical data on certain synthesised compounds:
**8a:** X=H, R₁=R₂= methyl (yield 57%)
   ¹H-NNM (CDCl₃): δ (ppm) 6.57-6.46 (m, 2H); 6.34 (s, 1H); 5.61-5.55 (dd, *J_{cis}*= 9.82, *J_{gem}*=1.85, 1H); 3.26 (s, 3H); 3.13 (s, 6H).
   m.p.:197°C dec
   MS: *m*/*z* 250 [M+Na]⁺; 477 [2M+Na]⁺.
**8b**: X=I, R₁=R₂= methyl (yield 57%)
   ¹H-NMR (CDCl₃): δ (ppm) 7.20-7.07 (dd, *J_{cis}*= 9.89, *J_{gem}*=6.76, 1H); 6.64-6.55 (d, *J=* 6.7, 1H); 5.71-5.65 (d, *J =* 9.89, 1H); 3.29 (s, 3H); 3.24 (s, 6H). m.p.:197°C dec.
   MS: *m*/*z* 354 [M+1]⁺; 376 [M+Na]⁺; 729 [2M+Na]⁺.

### EXAMPLE 9

Preparation of the compound **17**: 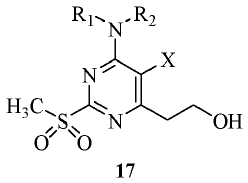 in which X = I, Cl, Br, H; R₁=R₂= methyl, ethyl, H; R₁= H and R₂= pentyl, propargyl, allyl.
a) react the product **16** (0.83 mmol) with *m*-chloroperbenzoic acid (2.47 mmol) in anhydrous CH₂Cl₂ (24 mL) and subject to magnetic stirring at ambient temperature for 12 hours;
b) evaporate the solvent at reduced pressure;
c) purify through chromatography on silica gel using a 95/5 CH₂Cl₂/MeOH mixture as an eluent.

Analytical data on certain synthesised compounds:
**17:** X=H, R₁=R₂= ethyl (yield 50%)
   ¹H-NNM (CDCl₃): δ (ppm) 6.35 (s, 1H); 4.74 (bs, 1H); 4.01-3.95 (t, *J =* 5.78, 2H); 3.52-3.44 (m, 4H); 3.23 (s, 3H); 2.91-2.85 (t, *J =* 5.78, 2H); 1.22-1.15 (t, *J*=6.8, 6H).
   MS: *m*/*z* 274 [M+1]⁺; 296 [M+Na]⁺; 312 [M+K]⁺.

### EXAMPLE 10

Preparation of the compound **18:** 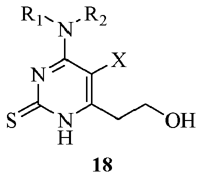 in which X = I, Cl, Br, H; R₁=R₂= methyl, ethyl, H; R₁= H and R₂= pentyl, propargyl, allyl.
a) react the product **17** (0.44 mmol) in a water solution of NaHS 1N (1 mL) and subject to magnetic stirring at reflux for 2 hours;
b) cool the solution, filtering it and evaporating under reduced pressure;
c) purify by chromatography on a silica gel column using a 9/1 CH₂Cl₂/MeOH mixture as an eluent.

Analytical data on certain synthesised compounds:
**18:** X=H, R₁=R₂= ethyl (yield 55%)
   ¹H-NNM (CD₃OD): δ (ppm) 6.12 (s, 1H); 3.82-3.70 (m, 3H); 3.55-3.44 (m, 4H); 2.68-2.62 (t, *J =* 6.06, 2H); 1.21-1.14 (t, *J*=7.79, 6H).
   MS: *m*/*z* 228 [M+1]⁺; 250 [M+Na]⁺; 476 [2M+Na]⁺.

### EXAMPLE 11

Preparation of the compound **19:** 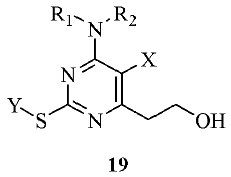 in which X = I, Cl, Br, H; R₁=R₂= methyl, ethyl, H; R₁= H and R₂ = pentyl, propargyl, allyl, Y= pentyl, *p*-methoxybenzyl.
a) react the product **18** (0.2 mmol) with the appropriate halogen derivative (0.2 mmol) and K₂CO₃ (0.2 mmol) in DMF (0.5 mL) in a closed test tube in a microwave oven (power max=300W) at 130 °C for 5 minutes;
b) dilute the solution with H₂O (3 mL) and extract 3 times with AcOEt (7 mL);
c) dry on anhydrous Na₂SO₄ and evaporate under reduced pressure;
d) purify the product through chromatography on silica gel using a 93/7 CH₂Cl₂/MeOH mixture as an eluent.

Analytical data on certain synthesised compounds:
**19a:** X=H, R₁=R₂= ethyl, Y=*p*-methoxybenzyl chloride (yield 95%)
   ¹H-NNM (CDCl₃): δ (ppm) 7.45-7.28 (m, 2H); 6.89-6.79 (m, 2H); 5.89 (s, 1H); 5.25 (s, 2H); 3.90-3.84 (t, *J*=5.6, 2H); 3.48-3.41 (m, 4H); 3.73 (s, 3H); 2.76-2.69 (t, *J*=5.6, 2H);
   1.21-1.11 (t, *J*=8.00, 6H).
   MS: *m*/*z* 348 [M+1]⁺; 370 [M+Na]⁺.
**19b:** X=H, R₁=R₂= ethyl, Y= pentyl (yield 95%)
   ¹H-NNM (CDCl₃): δ (ppm) 5.81 (s, 1H); 3.81-3.75 (t, *J*=5.10, 2H); 3.41-3.31 (q, *J*=6.88, 4H); 2.63-2.57 (t, *J*=5.10, 2H); 2.05-2.02 (m, 5H); 1.59-1.50 (m, 4H); 1.08-1.017 (t, *J*=6.88, 6H).
   MS: *m*/*z* 296 [M+1]⁺; 318 [M+Na]⁺.

### EXAMPLE 12

Preparation of the compound **6'**: 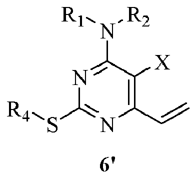 in which X = I, Cl, Br, H; R₁=R₂= methyl, ethyl, H; R₁= H and R₂= pentyl, propargyl, allyl, R₄= pentyl, *p*-methoxybenzyl.
a) react the compound **19** (0.1 mmol) in anhydrous dioxane (5 mL) in the presence of NaH (0.2 mmol) and leave at reflux for 3 hours;
b) bring to ambient temperature, filter and evaporate at reduced pressure.

**6'a :** X=H, R₁=R₂= ethyl, R₄=*p*-methoxybenzyl (yield 52%).
   ¹H-NMR (CDCl₃): δ (ppm) 7.34-7.29 (d, *J*=8.10, 2H); 6.89-6.85 (d, *J*=8.10, 2H); 6.82-6.78 (m, 2H); 5.98 (s, 1H); 5.53-5.47 (d, *J*=10.4, 1H); 4.60 (s, 1H); 3.87-3.76 (m, 5H); 1.19-1.11 (t, *J*=8.00, 6H).
   MS: *m*/*z* 330 [M+1]⁺; 352 [M+Na]⁺.
**6'b :** X=H, R₁=R₂= methyl, R₄=*p*-methoxybenzyl (yield 40%).
   ¹H-NMR (CDCl₃): δ (ppm) 7.35-7.31 (d, *J*=8.58, 2H); 6.81-6.77 (d, *J*=8.58, 2H); 6.59-6.45 (dd, *J*=17.31, *J*=9.83, 1H); 6.41-6.31 (dd, *J*=17.31, *J*=9.83, 1H); 6.01 (s, 1H); 5.50-5.44 (dd, *J*=9.83, *J*=2.15, 1H); 4.35 (s, 2H); 3.75 (s, 3H); 3.06 (s, 6H).
   MS: *m*/*z* 302 [M+1]⁺; 324 [M+Na]⁺.

### EXAMPLE 13

Preparation of the compound **9a:** 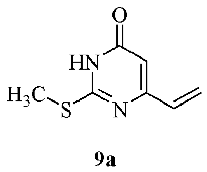
a) react the compound **13** (0.591 mmol) in DMF anhydrous (4.5 mL) in the presence of NaH (1 mmol) in a microwave oven (power max=300W) in a closed test tube, at 130 °C for 15 minutes, repeating irradiation twice;
b) dilute the reaction mixture in H₂O (3 mL) and extract 3 times with AcOEt (7 mL);
c) dry on anhydrous Na₂SO₄ and evaporate at reduced pressure;
d) purify through chromatography on silica gel using a 95/5 CH₂Cl₂/MeOH mixture as an eluent.

**9a**: yield 56%
   ¹H-NNM (CDCl₃): δ (ppm) 6.51-6.45 (m, 1H); 6.07 (s, 1H); 5.62-5.55 (m, 2H); 2.64 (s, 3 H).
   MS: *m*/*z* 191 [M+Na]⁺.

### EXAMPLE 14

Preparation of the compound **21:** 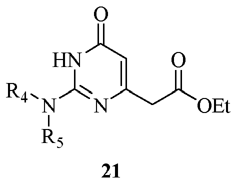
a) solubilise the compound **20** (2.19 mmol) in anhydrous CH₂Cl₂ (15 mL) and add *m-*chloroperbenzoic acid (6.57 mmol) solution in anhydrous CH₂Cl₂ (15 mL);
b) subj ect to magnetic stirring at ambient temperature for 3 hours;
c) add the appropriate amine (6.57 mmol) and subject to magnetic stirring at reflux for 12 hours;
d) wash the reaction mixture with a 1N solution of HCl, then with a saturate solution of Na₂CO₃ and with brine;
e) dry on anhydrous Na₂SO₄ and evaporate at reduced pressure;
f) purify through chromatography on silica gel using a 94/6 CH₂Cl₂/MeOH mixture as an eluent.

**21a:** R₄=H; R₅= benzyl; yield 68%
   ¹H-NNM (CDCl₃): δ (ppm) 7.32-7.26 (m, 5H); 5.44 (s, 1H); 4.55-4.53 (d, *J*=2.76, 2H);
   4.23-4.12 (q, *J*=7.08, 2 H); 3.37 (s, 2H); 1.25-1.19 (t, *J*=7.08, 3H),.
   MS: *m*/*z* 288 [M+1]⁺; 310 [M+Na]⁺ .

### EXAMPLE 15

### Preparation of the compound 26a

The 3-(4-methoxyphenyl)-propenol (0.3 mmol) is suspended in anhydrous DMF (1 mL) in the presence of trimethylphosphine (0.45 mL) (1 M solution in toluene) and left for 10 minutes. The reaction mixture is taken to 0 °C and CBr₄ (0.15 g, 0.45 mmol) is added. The mixture is irradiated in a microwave oven at 40 °C for 5 min and the appropriate thiouracil (0.3 mmol) is then added, synthesised as previously reported by Botta, Corelli et al. (J. Med. Chem. 2005, 48, 8000-8008). The mixture is irradiated at 130 °C for 5 min and is then diluted with water (2 mL) and extracted with diethyl ether (5 × 10 mL). Finally, the combined organic phases were dried on anhydrous Na₂SO₄ and evaporated. The combined organic phases are dried on anhydrous Na₂SO₄ and evaporated. The residue is purified by chromatographic flash to provide a solid that is then recrystallised.

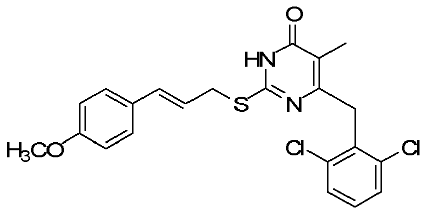

Yield 75%. Mp 211-212 °C. IR (CHCl₃) (v, cm⁻¹): 1539, 1660, 3002. ¹H NMR (DMSO-*d₆*): δ 2.03 (s, 3H), 3.47 (d, 2H, *J* = 7.20 Hz), 3.72 (s, 3H), 4.18 (s, 2H), 5.62-5.73 (m, 1H, *J* = 7.20 Hz, *Jₜᵣₐₙₛ* = 15.49 Hz), 6.24 (d, 1H, *Jₜᵣₐₙₛ* = 15.49 Hz), 6.85-6.89 (m, 2H), 7.13-7.44 (m, 5H). MS (ESI) *m*/*z*: 447 [M + H]⁺, 469 [M + Na]⁺. HPLC (C₈ column; CH₃OH/H₂O, 80/20) *t*_{R} 6.34 min.

### EXAMPLE 16

### Preparation of the compound 27a:

### Synthesis of the3-(4-Nitrophenyl)-propinol

Thep-nitroiodobenzene (2.14 mmol) and the propargyl alcohol are suspended in the minimum quantity of DMF. The following are added in order to this solution: triethylamine (4.28 mmol), PdCl₂(PPh₃)₂ (0.21) and CuI (0.65 mmol) the solution is subjected to magnetic stirring at ambient temperature for 5 minutes. It is diluted with water (2 mL) and it is extracted with ethyl acetate (3 × 10 mL). The combined organic phases are dried on anhydrous Na₂SO₄ and evaporated. The residue is purified by chromatographic flash to provide a solid that is then recrystallised. 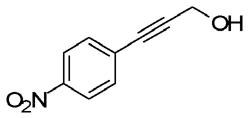 Yield 79%. Mp 95-96 °C. IR (CHCl₃) (v, cm⁻¹): 1522, 3031, 3609. ¹H NMR (CDCl₃): δ 4.52 (s, 2H), 7.55 (d, 2H, *Jₒᵣₜₕₒ* = 8.72 Hz), 8.16 (d, 2H, *Jₒᵣₜₕₒ* = 8.72 Hz). MS (ESI) *m*/*z*: 178 [M + H]⁺.

### 6-(2,6-Difluorobenzyl)-2-(3-(4-nitrophenyl)-propynylsulfanyl)-5-methylpyrimidine-4(3H)-one.

The 3-(4-Nitrophenyl)-propinol (0.3 mmol) is suspended in anhydrous DMF (1 mL) in the presence of trimethylphosphine (0.45 mL) (1 M solution in toluene) and left for 10 minutes. The reaction mixture is taken to 0 °C and CBr₄ (0.15 g, 0.45 mmol) is added. The mixture is irradiated in a microwave oven at 40 °C for 5 min and then the appropriate thiouracil (0.3 mmol) is added, synthesised as previously reported by Botta, Corelli et al. (J. Med. Chem. 2005, 48, 8000-8008). The mixture is irradiated at 130 °C for 5 min and is then diluted with water (2 mL) and extracted with diethyl ether (5 × 10 mL). Finally, the combined organic phases are dried on anhydrous Na₂SO₄ and evaporated. The combined organic phases are dried on anhydrous Na₂SO₄ and evaporated. The residue is purified by chromatographic flash to provide a solid that is then recrystallised. 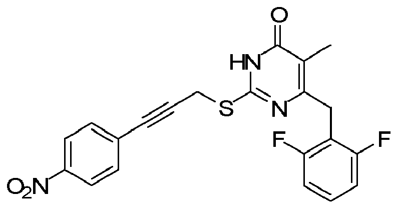 Yield 67%. IR (CHCl₃) (ν, cm⁻¹): 1643, 2931, 3368. ¹H NMR (DMSO-*d₆*): δ 2.02 (s, 3H), 3.93 (s, 4H), 6.95-6.99 (m, 1H), 7.15-7.35 (m, 2H), 7.51 (d, 2H, *Jₒᵣₜₕₒ* = 8.51 Hz), 8.18 (d, 2H, *Jₒᵣₜₕₒ* = 8.51 Hz). MS (ESI) *m*/*z*: 428 [M + H]⁺. HPLC (C₈ column; CH₃OH/H₂O, 80/20) *t*_{R} 4.10 min.

### EXAMPLE 17

### Preparation of the compound 28a:

### 1-(2-Bromoethoxymethyl)-4-methoxybenzene.

A solution of 2-bromoethanol (12.8 mmol) and K₂CO₃ (12.8 mmol) in anhydrous DMF (2 mL) is subjected to magnetic stirring at ambient temperature for 10 minutes. The 4-methoxybenzyl chloride (12.8 mmol) is added and is left at 60°C for 2h. It is diluted with water (2 mL) and is extracted with ethyl acetate (3 × 10 mL). The combined organic phases are dried on Na₂SO₄ anhydrous and evaporated. The residue is purified by chromatographic flash to provide a colourless oil.

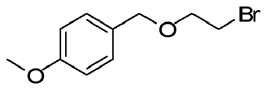

Yield 54%. ¹H NMR(CDCl₃): δ 3.45 (t, 2H, *J*= 5.83 Hz), 3.73 (t, 2H, *J*= 5.83 Hz), 3.85 (s, 3H), 4.55 (s, 2H), 6.85 (d, 2H, *Jₒᵣₜₕₒ* = 8.16 Hz), 7.25 (d, 2H, *Jₒᵣₜₕₒ* = 8.16 Hz).

### 6-(2,6-Dichlorobenzyl)-2-(2-(4-methoxybenzyloxy)-ethylsulfanyl)-5-methylpyrimidine-4(3H)-one.

The appropriate thiouracil (0.3 mmol) andl'1-(2-bromoettossimetil)-4-metossibenzene (0.3 mmol) are suspended in anhydrous DMF in the presence of K₂CO₃ (0.3 mmol), and the mixture is left at ambient temperature for 2h. It is diluted with water (2 mL) and is extracted with ethyl acetate (3 × 10 mL). The combined organic phases are dried on Na₂SO₄ anhydrous and evaporated. The residue is purified by chromatographic flash to provide a colourless oil. 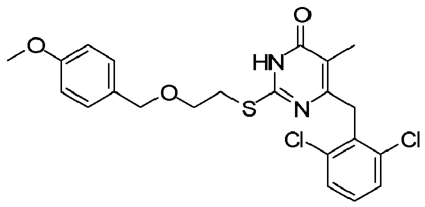 Yield 70%. Mp 138-140 °C. IR (CHCl₃) (v, cm⁻¹): 1644, 2958. ¹H NMR (DMSO-*d₆*): δ 1.99 (s, 3H), 2.85 (t, 2H, *J*= 5.82 Hz), 3.12 (t, 2H, *J*= 5.82 Hz), 3.71 (s, 3H), 4.10 (s, 2H), 4.20 (s, 2H), 6.83-6.87 (m, 2H), 7.12-7.36 (m, 5H), 12.51 (br s, 1H). MS (ESI) *m*/*z*: 465 [M + H]⁺, 487 [M + Na]⁺. HPLC (C₈ column; CH₃OH/H₂O, 80/20) *t*_{R} 6.24 min.

### BIOLOGICAL ACTIVITY

### Enzyme sample

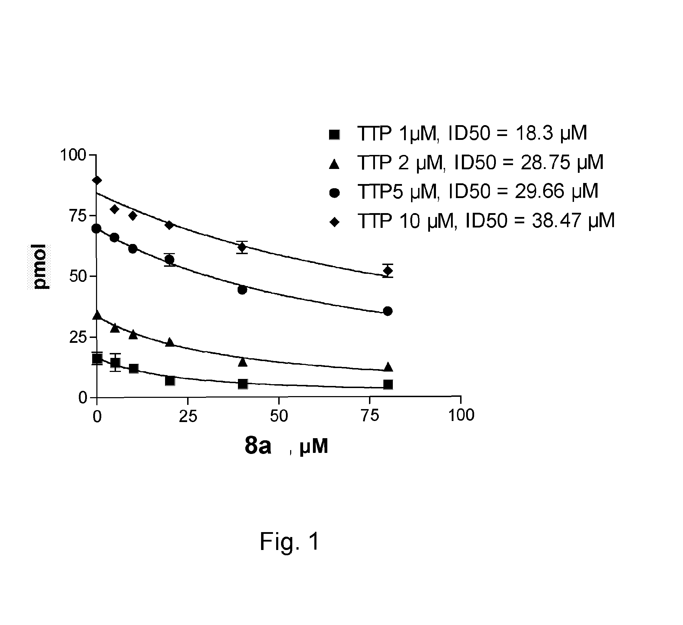

In a final volume of 25 microlitres the following reagents were mixed: 50 mM Tris-HCl pH 7.5, 0.25 mg/ml BSA, 0.5 mM DTT, 20 - 50 nM HIV-1 (recombinant) reverse transcriptase, nucleotide substrate dTTP marked radioactively (³H, 4 Ci/mmol) and nucleic acid (poly(rA)/oligo(dT)). The concentrations of dTTP and poly(rA)/oligo(dT) in standard conditions were 5 µM and 0.5 µM, respectively. In the experiments shown in Figures 1, 2 and 3, the concentrations of substrate were those indicated in the figures.

The reaction mixture is incubated 10 min. at 37°C and 20 µl are removed and deposited on a GF/C Whatman glass fibre filter measuring 25 mm in diameter. The filters are washed 3 times for 5 min. in 5% trichloroacetic acid and once for 5 min. in absolute alcohol. After being dried, the filters are immersed in a scintillating mixture (Packard) and the acid precipitable radioactivity is measured by a scintillation counter for β-emitting isotopes (Beckman).

In some experiments, the HIV-1 recombinant reverse transcriptase was substituted by a reverse transcriptase containing the mutations K103N and Y181I. They were produced by cloning the gene of the mutated RT in an expression vector for prokariotic cells according to the method reported in the literature (Maga G, Amacker M, Ruel N, Hubscher U, Spadari S. Resistance to nevirapine of HIV-1 reverse transcriptase mutants: loss of stabilizing interactions and thermodynamic or steric barriers are induced by different single amino acid substitutions. J Mol Biol. 1997 Dec 19;274(5):738-47).

The dose-response curves obtained by means of samples of enzyme activity in the presence of increasing doses of inhibitor were analysed according to the equation E(%) = Emax/(1+(I/ID₅₀), where E(%) is the fraction of enzyme activity that is measurable in the presence of each concentration of inhibitor, Emax is the activity in the absence of an inhibitor, I is the concentration of inhibitor. The ID₅₀ was calculated by analysing data using the GraphPad Prism graphic interpolation programme (for Macintosh).

### Test of inhibition of viral proliferation on culture cells

The biological activity of the compounds was evaluated on lymphoid cell lines MT-4 against the wild strain of HIV-1 NL4-3. The MT-4 cells were briefly infected with the appropriate HIV-1 or the infection was simulated to determine the cytoxicity, in the presence of different concentrations of potential inhibitor compounds. 5 days after the infection a colorimetric method was used that involves using a tetrazole salt (MTT) to evaluate the number of surviving cells. The mutant IRLL98 HIV-1 contains the following mutations in the coding sequence for RT:M41L, D67N, Y181C, M184V, R211K, T215Y (resistance to NRTI) and the mutations K101Q, Y181C, G190A (resistance to NNRTI). The HIV-1 mutants containing multi-NNRTI mutations, K103N or Y188L as mutants were supplied by the Medical Research Council Centralised Facility for AIDS Reagents, Herfordshire, UK

### BIOLOGICAL RESULTS

### Compound 8a

The compound **8a** proved to be the most active of the chemical series that we synthesised and has shown a reverse transcriptase ID₅₀ that is 3 orders of magnitude greater than that of the previously synthesised compound **MB3B** and a particular action mechanism compared with the compounds known hitherto in the literature.

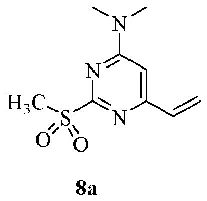

Studies of the action mechanism of the compound **8a** have shown that the molecular structure thereof is the binary complex RT:DNA and that inhibition of reverse transcriptase is competitive with the nucleotide. These characteristics are different from those of the classic non-nucleoside inhibitors, the target of which is the enzyme that is free of the substrates and the bond of which does not influence the subsequent link of the nucleic acid and nucleotide substrates.

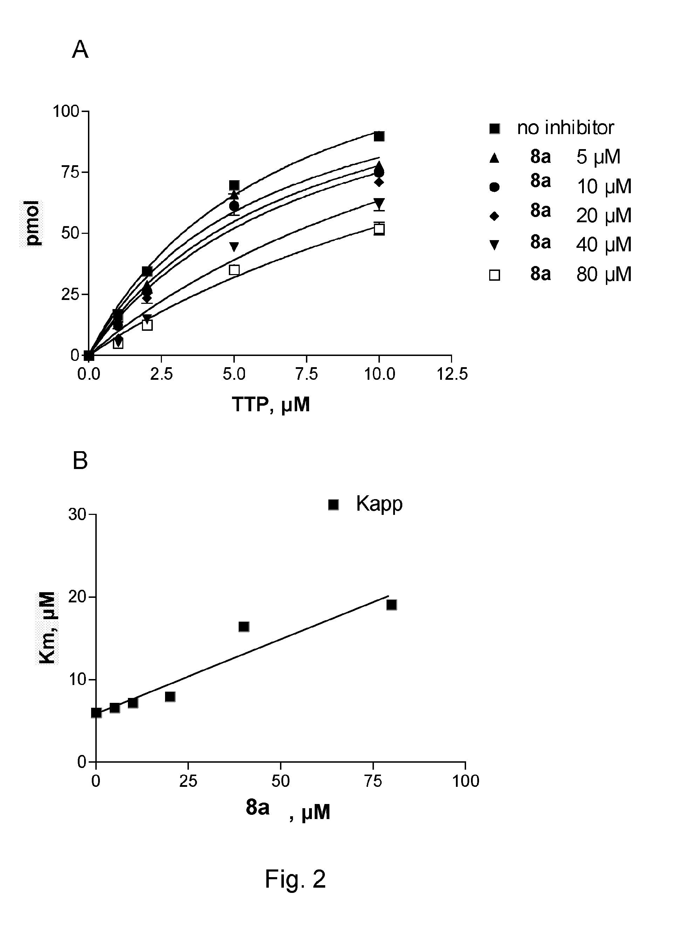

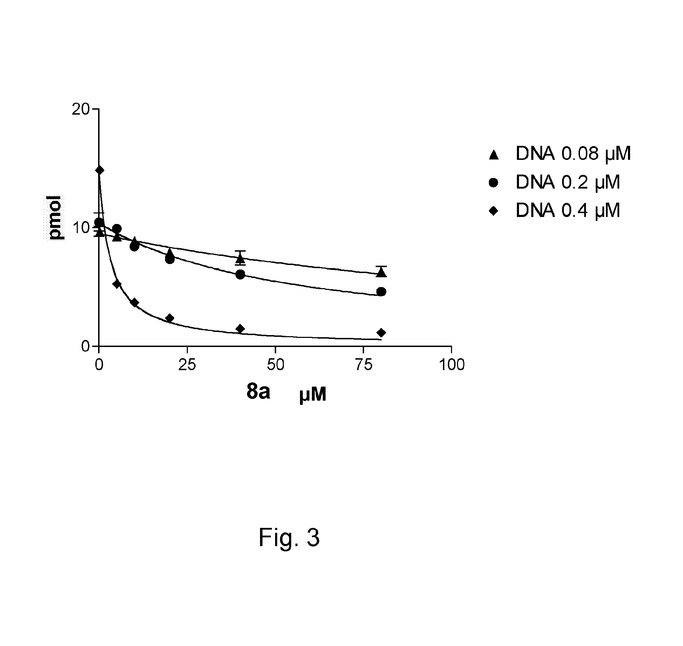

The demonstration of the action mechanism of **8a** is based on three distinct experimental observations:
1. The inhibition of the RT induced by **8a** decreases with the increase of the concentrations of TTP in the reaction. By performing titration of **8a** in a reverse transcription reaction *in vitro* with recombinant RT and in the presence of different concentrations of nucleotide substrate (in this case dTTP) a decrease in the capacity thereof to inhibit RT is shown, as is observed from the increase in the ID₅₀ values shown in Figure 1.
2. The apparent affinity (Kapp) of the RT for dTTP decreases as the concentration of **8a** increases. Equally if titrations of nucleotide substrate (dTTP) are taken in a reverse transcription reaction *in vitro* with recombinant RT and in the presence of different concentrations of **8a,** there is a reduction in the affinity of the RT for the substrate, as shown by the increase of apparent constant affinity (Kapp), in Figure 2. From these experiments, it can be concluded that the bond of **8a** and the nucleotide substrate with the RT is mutually exclusive, so that **8a** is a competitive inhibitor compared with the nucleotide substrate. From the enzyme analysis a competitive inhibition constant (Ki) has been calculated that is the equivalent of 15.3 µM.
3. The inhibition of the RT induced by **8a** increases the concentration of nucleic acid poly(rA)/oligo(dT) (generally known as DNA although it is an RNA-DNA hybrid). If titrations **8a** are made in a reverse transcription reaction *in vitro* with recombinant RT and in the presence of different concentrations of substrate (in this case poly(rA)/oligo(dT)) an increase of the capacity thereof to inhibit RT is observed, as shown by the progression of the curves shown in Figure 3

In view of the fact that RT binds the substrates in the order: 1) nucleic acid, 2) dNTP; with the increase in the concentration of DNA, the free enzyme becomes saturated with DNA and only afterwards does the binary complex RT:DNA bind to dNTP to form the ternary complex. The datum in Figure 3 thus suggests that **8a** preferentially binds to the binary complex RT:DNA. Alternatively, it could be hypothesised that **8a** complexes with the DNA and that the complex DNA:**8a** then binds to great affinity with the enzyme. Nevertheless, this does not seem probable, as **8a** is always present to excess in the reaction compared with DNA (**8a** is present in concentrations between 5 to 80 µM, whilst the DNA varies from 0.08 to 0.4 µM) and should therefore always be able to "titrate" the DNA.

It is thus possible to conclude that **8a** preferentially binds to the binary complex of the RT with the nucleic acid and that this bond prevents the subsequent interaction with the nucleotide substrate.

### Other derivatives

On the basis of the results obtained for **8a** other compounds have been synthesised that show an activity as inhibitors of the RT of HIV-1 and an action mechanism that is similar to **8a**. Table I shows the results of the enzyme tests conducted on some of the synthesised compounds.

**Table 1: Enzyme tests**

| | | | | | | | | **ID₅₀ (µM)** | | | **EC₅₀(mM)** | | | |
|---|---|---|---|---|---|---|---|---|---|---|---|---|---|---|
| **Comp** | **R₁** | **R₂** | **R₃** | **R₇** | **Z** | **X** | **Y** | **WT** | **K103N** | **Y1811** | **NL4-3 wt** | **K103N** | **Y181L** | **CC**_{**5**0} |
| **MB3B** | C₂H₅ | C₂H₅ | H | CH₃ | CH₂ | H | SO₂ | 21.3 | >400 | >400 | | | | |
| **8a** | CH₃ | CH₃ | H | CH₃ | CH₂ | H | SO₂ | 0.022 | 28 | 78 | >110 | >110 | >110 | 110 |
| **8b** | CH₃ | CH₃ | H | CH₃ | CH₂ | | SO₂ | 275 | >400 | >400 | >22.9 | >22.9 | >22.9 | 22.9 |
| **8'** | C₂H₅ | C₂H₅ | H | *p*-MeOBn | CH₂ H | | SO₂ | 467 | na | na | >42.9 | >42.9 | >42.9 | 42.9 |
| **6c** | CH₃ | CH₃ | H | CH₃ | CH₂ | H | S | >400 | | | >128 | >128 | >128 | 57.4 |
| **6a** | CH₃ | CH₃ | H | CH₃ | CH₂ | I | S | >400 | | | | | | |
| **6'** | C₂H₅ | C₂H₅ | H | *p*-MeOBn | CH₂ | H | S | na | na | na | >33.6 | 3.8 | >35.8 | 33.6 |
| **25a** | H | H | *p*-FPh | CH₃ | O | CH₃ | S | 2.1 | | | 0.58 | 5.96 | 0.21 | >90.2 |

As shown in Table 1, seven different positions (R1-R7, Z, X and Y) of the molecule of formula **I** have been considered in which R₆ = Y-R₇. The optimal position was found with the derivative **8a**, which shows an increase in activity in relation to the wild type enzyme of 2900 times. It is further also significantly active against enzymes containing significant resistance mutations in a clinical environment such as those in positions 103 and 181. On the other hand, the derivative **8a** did not show appreciable activity in tests of inhibition of viral proliferation in culture cells (Table 1). The activity of the compound **25a** at cell level is significantly worthy of note: not so much the inhibition of the wt as the maintenance of activity on mutated strains of the enzyme such as K103N and Y181L is of interest. It is these latter data that make the study of this new class of pyrimidine derivatives or further interest. As shown in Table 2, the compounds 26a-r generally have an excellent inhibiting activity against the wild-type strain of reverse transcriptase (WT), both in enzyme and cell tests.

**Table 2: Enzyme tests of the compounds 26a-r**

| **Comp** | **R13'** | **X** | **R₈** | **R₁'e R₅'** | **WT** | **K103N** | **Y181I** | **NL4-3 wt** | **K103N** | **Y181C** | **Y188L** | **CC50** |
|---|---|---|---|---|---|---|---|---|---|---|---|---|
| 26a | OCH₃ | CH₃ | H | Cl | 0.007 | 9 | >20 | 0.015 | >0.96 | >0.96 | >0.96 | 0.96 |
| 26b | NO₂ | CH₃ | H | Cl | 3.81 | >20 | >20 | >10.82 | >10.82 | >10.82 | >10.82 | >10.82 |
| 26c | CN | C₂H₅ | H | Cl | 0.188 | | na | 1.73 | >54.82 | 41% at 54.82 | >54.82 | >54.82 |
| 26d | N(CH₃)₂ | CH₃ | H | Cl | 0.778 | | na | >54.35 | >54.35 | >54.35 | >54.35 | >54.35 |
| 26e | OCH₃ | C₂H₅ | H | Cl | 0.056 | >20 | >20 | 0.0015 | >0.19 | >0.19 | >0.19 | 0.19 |
| 26f | NO₂ | C₂H₅ | H | Cl | 2.5 | >20 | >20na | | | | | |
| 26g | OCH₃ | CH₃ | CH₃ | Cl | 0.018 | >20 | >20 | 0.009 | 2.47 | 3.6 | 1.26 | >10.84 |
| 26h | NO₂ | CH₃ | CH₃ | Cl | 0.102 | >20 | >20 | | | | | |
| 26i | NO₂, | CH₃ | H | F | 11.88 | >20 | >20 | 0.26 | >11.65 | >11.65 | >11.65 | 11.65 |
| 261 | OCH₃ | CH₃ | H | F | 0.25 | >20 | >20 | 0.043 | >3.93 | >3.93 | >3.93 | 3.93 |
| 26m | NO₂ | CH₃ | CH3 | F | 0.283 | >20 | >20 | 0.14 | >11.28 | >11.28 | >11.28 | >11.28 |
| 26n | OCH₃ | CH₃ | CH₃ | F | 0.292 | 3.7 | >20 | 0.0002 | 0.26 | 0.13 | 0.07 | >11.7 |
| 26° | CN | CH₃ | CH₃ | F | 0.161 | | na | 0.14 | >59.1 | >59.1 | >59.1 | >59.1 |
| 26p | OCH₃ | C₂H₅ | CH₃ | F | 0.016 | 0.4 | >20 | 0.0002 | 1.02 | 0.70 | 0.63 | 5.54 |
| 26q | NO₂ | C₂H₅ | CH₃ | F | 0.009 | >20 | >20 | 0.0004 | >5.75 | >5.75 | 1.94 | 5.75 |
| 26r | CN | C₂H₅ | CH₃ | F | 0.048 | | na | 0.59 | >27.23 | >27.23 | >27.23 | 27.23 |

| | | | | | | | | | | | | |
|---|---|---|---|---|---|---|---|---|---|---|---|---|
| R₁₁', R₁₂', R₁₄', R₁₅', R₂', R₃', R₄'= H | | | | | | | | | | | | |

In particular, the compounds 26a, 26e, 26g, 261, 26n, 26p, 26q showed nanomolar and subnanomolar activity. As far as the activity of the compounds 26a-r on mutant strains is concerned, enzyme tests have enabled lead compounds to be identified in the compounds 26a, 26n, 26p, which lead compounds are interesting for designing further active compounds on the mutant K103N. These compounds in fact showed micromolar and submicromolar activity in cell tests on both the mutant K103N and on the other mutants examined (Y181C, Y188L). The compounds 27a-c shown in Table 3, which respectively show the similar ones of the compounds 26i, 26g and 26a performing an alkyne rather than an alkene function on the lateral chain bound to the C2 of the pyrimidinone core, showed a decrease in inhibitory activity in both cell and enzyme tests.

**Table 3: Enzyme tests on the compounds 27a-c**

| **Comp.** | **R₁₄'** | **x** | **R₈₈** | **R₁-Rₛ'** | **WT** | **NL4-3wt** | **K103N** | **Y181C** | **Y188L** | **CC50** |
|---|---|---|---|---|---|---|---|---|---|---|
| 27a | NO₂ | CH₃ | H | F | 0.416 | 40.09 | >58.55 | >58.55 | >58.55 | >58.55 |
| 27b | OCH₃ | CH₃ | CH₃ | Cl | | 0.50 | >54.47 | 33.55 | 24.84 | >57.47 |
| 27c | OCH₃ | CH₃ | H | Cl | | 2.47 | >56.18 | 40% at 56.18 | >56.18 | >56.18 |

| | | | | | | | | | | |
|---|---|---|---|---|---|---|---|---|---|---|
| R₁₁', R₁₂', R₁₄', R₁₅', R₂', R₃', R₄' = H | | | | | | | | | | |

This decrease in activity can thus be correlated to the loss of important interactions with the aromatic portion bound to the lateral chain in C2 following the introduction of a planar linear system like the alkyl system.

## Claims

1. A compound of formula V: 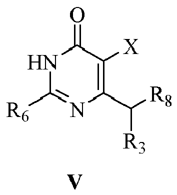 in which:
X represents H, I, Cl, Br, methyl, ethyl, propyl or alkyl, aryl or aralkyl substituted groups;
R₃ represents an aryl with the formula: 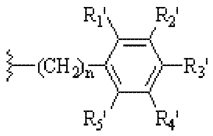 in which R₁', R₂', R₃', R₄', R₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups, n is comprised between 0 and 4;
R₆ represents Y-R₇, 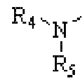 in which:
Y = S, SO or SO₂,
R₇ represents methyl, ethyl, propyl, butyl, pentyl, cyclopentyl, cyclohexyl, aryl substituted groups with the formula: 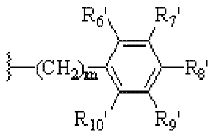 in which R₆', R₇', R₈', R₉', R₁₀' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups, m is comprised between 0 and 4;
R₇ also represents substituted cinnamoils with the formula: 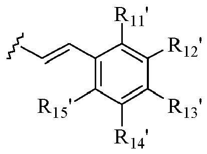 in which R₁₁', R₁₂', R₁₃', R₁₄', R₁₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)_{2,} NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups;
R₇ also represents substituted systems with the formula: 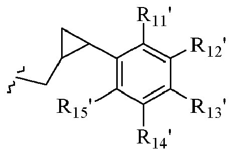 in which R₁₁', R₁₂', R₁₃', R₁₄', R₁₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups;
R₇ also represents substituted systems with the formula: 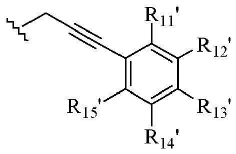 in which R₁₁', R₁₂', R₁₃', R₁₄', R₁₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups;
R₇ also represents substituted systems with the formula: 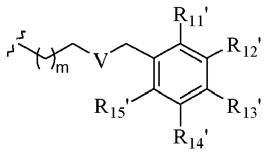 in which V is N, S, O and R₁₁', R₁₂', R₁₃', R₁₄', R₁₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups, m is comprised between 0 and 4;
R₄ and R₅ represent independently H, methyl, ethyl, benzyl, cyclopentyl, allyl, propargyl, pentyl, aryl substituted groups with the formula: 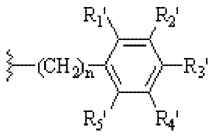 in which R₁', R₂', R₃', R₄', R₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups, n is comprised between 0 and 4;
R₈ represents H or methyl;
or a pharmaceutically acceptable salt, prodrug, stereoisomers or tautomer thereof,
wherein if X = ethyl,
- R₃ = 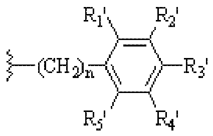 in which n = 0, R_{1'} = R_{5'} = Cl or F, R_{2'}=R_{3'}=R_{4'}= H, R₆ = Y-R₇ in which Y = S and R₇ is a substituted cinnamoils with the formula: 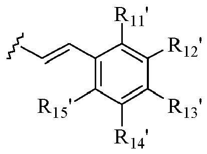 in which R₁₁', R₁₂' , R₁₄', R₁₅' = H, and R₁₃' = CN or OCH₃ or NO₂ and R₈ = H; wherein if R₈ = H
- X = methyl, R₃ represents an aryl with the formula: 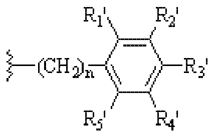 in which n = 0, R_{1'} = R_{5'} = Cl, R_{2'}=R_{3'}=R_{4'}= H, R₆ = Y-R₇ in which Y = S and R₇ is a substituted cinnamoils with the formula: 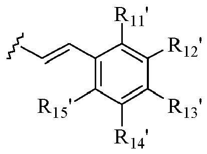 in which R₁₁', R_{12'} , R_{14'}, R_{15'} = H, and R_{13'} = OCH₃, NO₂ or N(CH₃)₂; or
- X = methyl, R₃ represents an aryl with the formula: 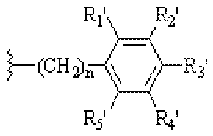 in which n = 0, R_{1'} = R_{5'} = F, R_{2'}=R_{3'}=R_{4'}= H, R₆ = Y-R₇ in which Y = S and R₇ is a substituted cinnamoils with the formula: 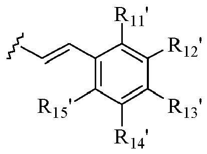 in which R₁₁', R₁₂', R₁₄', R₁₅' = H, and R_{13'} = OCH₃ or NO₂; or
- X = methyl, R₃ represents an aryl with the formula: 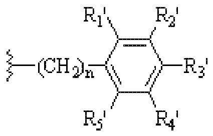 in which n = 0, R_{1'} = R_{5'} = F, R_{2'}=R_{3'}=R_{4'}= H, R₆ = Y-R₇ in which Y = S and R₇ is a substituted system with the formula: 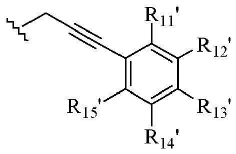 in which R₁₁', R₁₂', R_{14'}, R_{15'} = H and R_{13'}= NO₂; or
- X = methyl, R₃ represents an aryl with the formula: 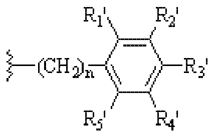 in which n = 0, R_{1'} = R_{5'} = Cl, R_{2'}=R_{5'}=R_{4'}= H, R₆ = Y-R₇ in which Y = S and R₇ is a substituted system with the formula: 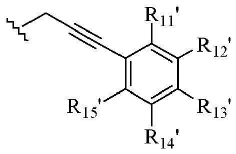 in which R₁₁', R₁₂', R_{14'}, R₁₅' = H and R₁₃'= OCH₃; or
- X = methyl, R₃ represents an aryl with the formula: 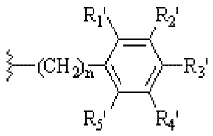 in which n = 0, R₁' = R₅' = Cl, R₂'=R₃'=R₄'= H, R₆ = Y-R₇ in which Y = S and R₇ is a substituted system with the formula: 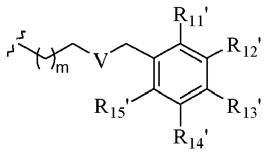 in which m is 1, V is O and R₁₁', R₁₂', R₁₄', R₁₅' are H and R₁₃'= OCH₃.

2. A compound according to claim 1 in which:
X represents H, I, Cl, Br, methyl, ethyl, propyl or alkyl, aryl or aralkyl substituted groups;
R₃ represents an aryl with the formula: 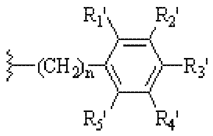 in which R₁', R₂', R₃', R₄', R₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁-₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups, n is comprised between 0 and 4;
R₆ represents Y-R₇, in which:
Y = S, SO or SO₂,
R₇ represents substituted cinnamoils with the formula: 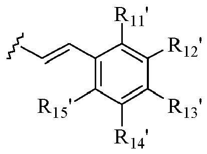 in which R₁₁', R₁₂', R₁₃', R₁₄', R₁₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups;
R₇ also represents substituted systems with the formula: 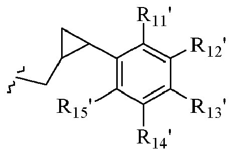 in which R₁₁', R₁₂', R₁₃', R₁₄', R₁₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups;
R₇ also represents substituted systems with the formula: 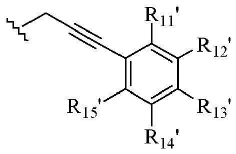 in which R₁₁', R₁₂', R₁₃', R₁₄', R₁₅' are independently H, C₁₋₆alkyl, C₂₋₆alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups;
R₇ also represents substituted systems with the formula: 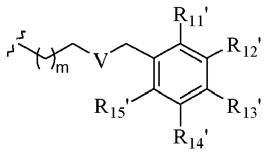 in which V is N, S, O and R₁₁', R₁₂', R₁₃', R₁₄', R₁₅' are independently H, C₁₋₆alkyl, C₂₋₆ alkenyl, C₂₋₆alkynyl, aryl substituted groups, halo, haloalkyl (in particular CF₃), OCH₃, NO₂, CN, CONH₂, CONH-C₁₋₆alkyl, CON(C₁₋₆alkyl)₂, NH₂, NH-C₁₋₆alkyl, N(C₁₋₆alkyl)₂, NHC(O)alkyl, NHSO₂-C₁₋₆alkyl, SO₂NH₂, SO₂NHC₁₋₆alkyl, SO₂N(C₁₋₆alkyl)₂, OZ' or SZ' where Z' is H, or alkyl, aryl or aralkyl substituted groups, m is comprised between 0 and 4;
R₈ represents H or methyl;
or a pharmaceutically acceptable salt, prodrug, stereoisomers or tautomer thereof,
wherein if X = ethyl,
- R₃ = 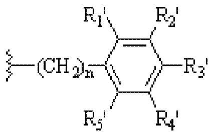 in which n = 0, R_{1'} = R_{5'} = Cl or F, R_{2'}=R_{3'}=R_{4'}= H, R₆ = Y-R₇ in which Y = S and R₇ is a substituted cinnamoils with the formula: 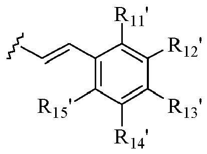 in which R₁₁', R₁₂' , R₁₄', R₁₅' = H, and R₁₃' = CN or OCH₃ or NO₂ and R₈ = H;
wherein if R₈ = H
- X = methyl, R₃ represents an aryl with the formula: 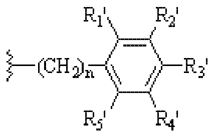 in which n = 0, R_{1'} = R_{5'} = Cl, R_{2'}=R_{3'}=R_{4'}= H, R₆ = Y-R₇ in which Y = S and R₇ is a substituted cinnamoils with the formula: 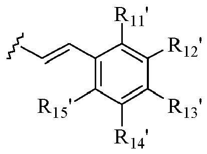 in which R₁₁', R₁₂' , R₁₄', R₁₅' = H, and R₁₃' = OCH₃, NO₂ or N(CH₃)₂; or
- X = methyl, R₃ represents an aryl with the formula: 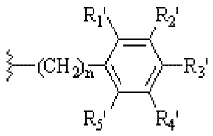 in which n = 0, R_{1'} = R_{5'} = F, R_{2'}=R_{3'}=R_{4'}= H, R₆ = Y-R₇ in which Y = S and R₇ is a substituted cinnamoils with the formula: 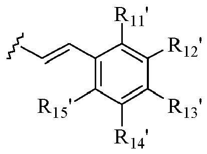 in which R₁₁', R₁₂' , R₁₄', R₁₅' = H, and R₁₃' = OCH₃ or NO₂; or
- X = methyl, R₃ represents an aryl with the formula: 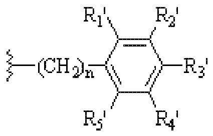 in which n = 0, R_{1'} = R_{5'} = F, R_{2'}=R_{3'}=R_{4'}= H, R₆ = Y-R₇ in which Y = S and R₇ is a substituted system with the formula: 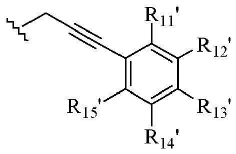 in which R₁₁', R₁₂', R_{14'}, R₁₅' = H and R₁₃'= NO₂; or
- X = methyl, R₃ represents an aryl with the formula: 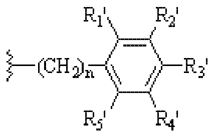 in which n = 0, R_{1'} = R_{5'} = Cl, R_{2'}=R_{3'}=R_{4'}= H, R₆ = Y-R₇ in which Y = S and R₇ is a substituted system with the formula: 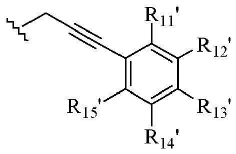 in which R₁₁', R₁₂', R_{14'}, R₁₅' = H and R₁₃'= OCH₃; or
- X = methyl, R₃ represents an aryl with the formula: 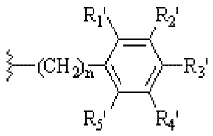 in which n = 0, R₁' = R₅' = Cl, R₂'=R₃'=R₄'= H, R₆ = Y-R₇ in which Y = S and R₇ is a substituted system with the formula: 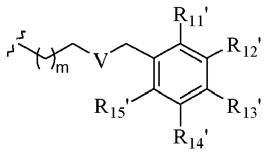 in which m is 1, V is O and R₁₁', R₁₂', R₁₄', R₁₅' are H and R₁₃'= OCH₃.

3. A compound according to claims 1 or 2 for medical use.

4. A compound according to any of preceeding claims for use as an anti-viral agent.

5. The compound according to claim 4 wherein the anti-viral activity is anti-HIV.

6. Pharmaceutical composition comprising a pharmaceutically effective and acceptable quantity of the compound according to claims 1 or 2 and suitable dilutants.

7. The pharmaceutical composition according to claim 6 further comprising at least another compound having an anti-HIV activity.

8. Method for the preparation of a compound of general formula V according to claim 1 or 2, comprising the phases shown in diagrams 6, 7 or 8.
